# EUROPEAN PATENT APPLICATION

(11) **EP 4 368 178 A1**
(43) Date of publication of application: **15.05.2024**
(21) Application number: 23209051.4
(22) Date of filing: 10.11.2023
(51) Int. Cl.: A61K 31/191, A61K 35/00, A23L 21/25, A61K 45/06, A61P 11/02, A61P 17/00, A61P 27/02, A61P 31/00, A61P 31/02, A61K 35/64

(54) **COMPOSITIONS OF HONEY AND GLUCONIC ACID AND USES THEREFOR**

(30) Priority: 10.11.2022 AU 2022903374
(71) Applicant: Melcare Medical Pty Ltd, Mount Cotton, QLD 4165 (AU)
(72) Inventor: MOLONEY, Anthony Peter, Mount Cotton, 4165 (AU)
(74) Representative: Vossius & Partner Patentanwälte Rechtsanwälte mbB

(57) **Abstract**

Disclosed are compositions comprising honey or a honey analogue, gluconic acid or a pharmaceutically acceptable salt or derivative thereof and a pharmaceutically acceptable carrier, and their use for treating or inhibiting the development of an infection or an inflammatory condition or for treating a wound. Also disclosed is the use of gluconic acid or a pharmaceutically acceptable salt or derivative thereof for stabilising the pH of a composition comprising honey or a honey analogue.

## Description

### RELATED APPLICATIONS

This application claims priority to Australian Provisional Patent Application No. 2022903374 entitled "Compositions and Uses Therefor" filed 10 November 2022, the contents of which are incorporated herein by reference in their entirety.

### FIELD OF THE INVENTION

This invention relates generally to compositions comprising honey or a honey analogue, gluconic acid or a pharmaceutically acceptable salt or derivative thereof and a pharmaceutically acceptable carrier, and their use for treating or inhibiting the development of an infection or an inflammatory condition or for treating a wound. The invention also relates to the use of gluconic acid or a pharmaceutically acceptable salt or derivative thereof for stabilising the pH of a composition comprising honey or a honey analogue. Further, the present invention relates to methods for stabilising the pH of a composition comprising honey or a honey analogue, wherein the method comprises adding gluconic acid or a pharmaceutical acceptable salt or derivative thereof to said composition.

### BACKGROUND OF THE INVENTION

The reference in this specification to any prior publication (or information derived from it), or to any matter which is known, is not, and should not be taken as an acknowledgment or admission or any form of suggestion that that prior publication (or information derived from it) or known matter forms part of the common general knowledge in the field of endeavour to which this specification relates.

Honey has been known for many years to have beneficial therapeutic properties. Therapeutic interest in honey has increased in more recent years due to the demonstration of beneficial effects of raw honey in wound care, which are likely due to the pharmacological and antimicrobial properties, particularly in certain types of honey. Several factors have been proposed as being linked to the antimicrobial activity of honey, including a high sugar content and low water activity, a pH in the range of from 3.2 to 4.8, the presence of glucose oxidase which contributes to hydrogen peroxide generation and other less understood plant derived factors.

While all honey is known to have healing properties, particular types of honey are known to have broader antimicrobial activity than other types of honey. In this regard, honey sourced from the Myrtacaea family, in particular *Leptospermum* species, is known to have a higher non-peroxide antimicrobial activity than other types of honey. This activity is also retained following exposure to moderate heat, light and gamma radiation.

The acidic pH of honey has been proposed to play a role in its therapeutic activity and is associated with many properties of honey, including the texture and stability of the honey itself. While beneficial for the activity and properties of honey, an acidic pH can be problematic for therapeutic applications, particularly for ophthalmic or intranasal use or use in wound treatment. In this regard, an acidic pH can lead to an initial stinging sensation and discomfort upon administration and low patient compliance. As a result, the pH of therapeutic honey compositions is required to be closely controlled to balance patient comfort and compliance with the maintenance of the therapeutic activity and beneficial properties of the honey.

The pH of honey compositions is also known to fluctuate over time, with compositions increasing in acidity during storage. This acidic change in pH is also known to be accelerated by particular storage conditions, including increased temperatures, and increased dilution of the honey. As a result, therapeutic honey compositions often have a short shelf-life, especially ophthalmic compositions.

The complex chemical composition of honey and the criticality of the components and pH for the therapeutic activity has made preparing therapeutic honey compositions with extended stability under typical storage conditions challenging. Therapeutically active honey compositions with improved pH stability and an extended shelf-life are desired.

### SUMMARY OF THE INVENTION

The present invention is predicated in part on the discovery that the addition of gluconic acid or a salt thereof reduces fluctuations in the pH of compositions comprising honey over an extended period of time. The minimal change in pH over time is beneficial for the shelf-life of compositions comprising honey or a honey analogue, especially compositions for therapeutic use, such as ocular use, wherein compositions with increased acidity can cause patient discomfort upon administration. As such, the inventor has conceived that gluconic acid, or a pharmaceutically acceptable salt or derivative thereof can be added to compositions comprising honey or a honey analogue and a pharmaceutically acceptable carrier to stabilise the pH of the composition. The inventor has also conceived that compositions comprising honey or a honey analogue, gluconic acid or a pharmaceutically acceptable salt or derivative thereof and a pharmaceutically acceptable carrier may be used for treating or inhibiting the development of an infection or an inflammatory condition, or for treating a wound.

Accordingly, in one aspect, there is provided a composition comprising honey or a honey analogue, gluconic acid or a pharmaceutically acceptable salt or derivative thereof and a pharmaceutically acceptable carrier.

In some embodiments, the honey or honey analogue is in an amount in the range of from about 2% to about 98% w/w (and all one tenth integer percentages in between), about 2% to about 50% w/w, about 2% to about 45% w/w or about 7% to about 18% w/w. In exemplary embodiments, the honey or honey analogue is in an amount of about 16.5% w/w.

In some embodiments, the gluconic acid or a pharmaceutically acceptable salt or derivative thereof is in an amount in the range of from about 0.05% to about 10% w/w (and all one tenth integer percentages in between), about 0.1% to about 5% w/w, about 0.1% to about 3% w/w, about 0.1% to about 1% w/w, or about 0.1% to about 0.5% w/w. In particular embodiments, the gluconic acid or a pharmaceutically acceptable salt or derivative thereof is in an amount of about 0.3% w/w.

In specific embodiments, the gluconic acid is in the form of a pharmaceutically acceptable salt, such as a pharmaceutically acceptable salt selected from the group consisting of sodium gluconate, calcium gluconate, potassium gluconate, magnesium gluconate, zinc gluconate, copper gluconate and ferrous gluconate. In exemplary embodiments, the pharmaceutically acceptable salt is sodium gluconate.

The composition may further comprise a preservative, such as a preservative selected from the group consisting of benzoic acid, sorbic acid, a flavonoid, a phenolic acid, abscisic acid, ascorbic acid and pharmaceutically acceptable salts and combinations thereof. In specific embodiments, the preservative is benzoic acid or a pharmaceutically acceptable salt thereof, such as sodium benzoate.

In some embodiments, the pH of the composition is in the range of from about 3.5 to about 4.5 (and all one tenth integers in between), especially about 3.9 to about 4.1.

While any composition is contemplated, in particular embodiments, the composition is a pharmaceutical composition. In certain embodiments, the composition is in the form of a nasal spray, nasal lavage, eye drop, eye spray, eye wash, eye ointment, ear wash, ear drop, throat spray, douche, wound dressing, wound dressing hydrator, wound cleansing solution or lung aspirant; especially a nasal spray or an eye drop.

In some embodiments, the carrier is an aqueous carrier (e.g. saline or water), glycerol or a combination thereof.

In particular embodiments, the composition comprises honey. The invention contemplates the use of any type of honey, including honey substantially sourced from *Leptospermum* species. In particular embodiments, the honey is substantially sourced from *Leptospermum* species, such as a species selected from the group consisting of *Leptospermum scoparium, Leptospermum polygalifolium, Leptospermum semibaccatum, Leptospermum trinervium, Leptospermum whitei, Leptospermum speciosum, Leptospermum liversidgei* and combinations thereof.

The composition may further comprise an ocular lubricant, such as a lubricant selected from the group consisting of glucose, glycerol, polyethylene glycol, propylene glycol and combinations thereof; especially glycerol.

In another aspect, there is provided a method of treating or inhibiting the development of an infection in a subject, comprising administering a composition of the invention to the subject.

In particular embodiments, the infection is a bacterial or viral infection, especially a viral infection. The infection may be an acute or chronic infection.

In some embodiments, the infection is an ophthalmic infection, vaginal infection, nasal infection, ear infection, lung infection, sinus infection, throat infection, skin infection or wound infection; especially an ophthalmic infection or a nasal infection.

Further provided, in another aspect, is a method of treating or inhibiting the development of an inflammatory condition in a subject, comprising administering a composition of the invention to the subject.

In some embodiments, the inflammatory condition is an ocular condition, such as blepharitis, dry eye syndrome or corneal oedema.

In alternative embodiments, the inflammatory condition is a nasal condition, such as rhinitis. In specific embodiments, the inflammatory condition is sinusitis.

In alternative embodiments, the inflammatory condition is eczema, contact dermatitis or urticaria.

Also contemplated is where the inflammatory condition is an allergic reaction, such as an allergic reaction is associated with an insect bite or an infection.

In specific embodiments, the inflammatory condition is associated with an infection.

Also provided, in another aspect, is a method for stabilising the pH of a composition comprising honey or a honey analogue, wherein the method comprises adding gluconic acid or a pharmaceutically acceptable salt or derivative thereof to the composition.

Further provided, in another aspect, is the non-pharmaceutical use of gluconic acid or a pharmaceutically acceptable salt or a derivative thereof for stabilising the pH of a composition comprising honey or a honey analogue.

The method or the use may further comprise adding a carrier to the composition before or after adding the gluconic acid or a pharmaceutically acceptable salt or derivative thereof.

In particular embodiments, the honey or honey analogue is in an amount in the range of from about 2% to about 98% w/w (and all one tenth integer percentages in between), about 2% to about 50% w/w or about 2% to about 45% w/w or about 7 to about 18% w/w. In exemplary embodiments, the honey or honey analogue is in an amount of about 16.5% w/w.

In some embodiments, the gluconic acid or a pharmaceutically acceptable salt or derivative thereof is added in an amount in the range of from about 0.05% to about 10% w/w of the composition (and all one tenth integer percentages in between), about 0.1% to about 5% w/w of the composition, about 0.1% to about 3% w/w of the composition, about 0.1% to about 1% w/w of the composition or about 0.1% to about 0.5% w/w of the composition. In specific embodiments, the gluconic acid or a pharmaceutically acceptable salt or derivative thereof is added in an amount of about 0.3% w/w of the composition.

In particular embodiments, the gluconic acid is in the form of a pharmaceutically acceptable salt, such as sodium gluconate, calcium gluconate, potassium gluconate or magnesium gluconate, especially sodium gluconate.

In some embodiments, the method or the use further comprises adding a preservative to the composition before or after adding the gluconic acid or a pharmaceutically acceptable salt or derivative thereof. In specific embodiments, the preservative is selected from the group consisting of benzoic acid, sorbic acid, a flavonoid, a phenolic acid, abscisic acid, ascorbic acid and pharmaceutically acceptable salts and combinations thereof; especially benzoic acid or a pharmaceutically acceptable salt thereof, such as sodium benzoate.

In some embodiments, the pH of the composition is maintained in the range of from about 3.5 to about 4.5 (and all one tenth integers in between), especially about 3.9 to about 4.1. In particular embodiments, the pH of the composition is maintained for a period of at least six months, especially a period of at least 2 years.

In some embodiments, the composition is a pharmaceutical composition. In exemplary embodiments, the composition is in the form of a nasal spray, nasal lavage, eye drop, eye spray, eye wash, eye ointment, ear wash, ear drop, throat spray, douche, wound dressing, wound dressing hydrator, wound cleansing solution or lung aspirant; especially a nasal spray or an eye drop.

In particular embodiments, the carrier is an aqueous carrier such as saline or water, glycerol or a combination thereof.

In particular embodiments, the composition comprises honey. While the use of any honey is contemplated, in some embodiments, the honey is substantially sourced from *Leptospermum* species, such as *Leptospermum scoparium, Leptospermum polygalifolium, Leptospermum semibaccatum, Leptospermum trinervium, Leptospermum whitei, Leptospermum speciosum, Leptospermum liversidgei* and combinations thereof.

In some embodiments, the method or the use further comprises adding an ocular lubricant to the composition before or after adding the gluconic acid or a pharmaceutically acceptable salt or derivative thereof. In exemplary embodiments, the ocular lubricant is selected from the group consisting of glucose, glycerol, polyethylene glycol, propylene glycol and combinations thereof; especially glycerol.

### DETAILED DESCRIPTION OF THE INVENTION

### 1. Definitions

Unless defined otherwise, all technical and scientific terms used herein have the same meaning as commonly understood by those of ordinary skill in the art to which the invention belongs. Although any methods and materials similar or equivalent to those described herein can be used in the practice or testing of the present invention, preferred methods and materials are described. For the purposes of the present invention, the following terms are defined below.

The articles "a" and "an" are used herein to refer to one or to more than one (i.e. to at least one) of the grammatical object of the article. By way of example, "an element" means one element or more than one element.

By "about" is meant a quantity, level, value, number, frequency, percentage, dimension, size, amount, weight or length that varies by as much 15, 14, 13, 12, 11, 10, 9, 8, 7, 6, 5, 4, 3, 2 or 1 % to a reference quantity, level, value, number, frequency, percentage, dimension, size, amount, weight or length.

The terms "administration concurrently" or "administering concurrently" or "co-administering" and the like refer to the administration of a single composition containing two or more agents, or the administration of each agent as separate compositions and/or delivered by separate routes either contemporaneously or simultaneously or sequentially within a short enough period of time that the effective result is equivalent to that obtained when all such agents are administered as a single composition. By "simultaneously" is meant that the agents are administered at substantially the same time, and desirably together in the same composition. By "contemporaneously" it is meant that the agents are administered closely in time, e.g., one agent is administered within from about one minute to within about one day before or after another. Any contemporaneous time is useful. However, it will often be the case that when not administered simultaneously, the agents will be administered within about one minute to within about eight hours and suitably within less than about one to about four hours. When administered contemporaneously, the agents are suitably administered at the same site on the subject. The term "same site" includes the exact location, but can be within about 0.5 to about 15 centimeters, preferably from within about 0.5 to about 5 centimeters. The term "separately" as used herein means that the agents are administered at an interval, for example at an interval of about a day to several weeks or months. The agents may be administered in either order. The term "sequentially" as used herein means that the agents are administered in sequence, for example at an interval or intervals of minutes, hours, days or weeks. If appropriate the agents may be administered in a regular repeating cycle.

The term "agent" includes a compound that induces a desired pharmacological and/or physiological effect. The term also encompasses pharmaceutically acceptable and pharmacologically active ingredients of those compounds specifically mentioned herein including but not limited to salts, esters, amides, prodrugs, active metabolites, analogs and the like. When the above term is used, then it is to be understood that this includes the active agent *per se* as well as pharmaceutically acceptable, pharmacologically active salts, esters, amides, prodrugs, metabolites, analogues, etc. The term "agent" is not to be construed narrowly but extends to small molecules, proteinaceous molecules such as peptides, polypeptides and proteins as well as compositions comprising them and genetic molecules such as RNA, DNA and mimetics and chemical analogs thereof as well as cellular agents.

As used herein, the term "and/or" refers to and encompasses any and all possible combinations of one or more of the associated listed items, as well as the lack of combinations when interpreted in the alternative (or).

As used herein, the term "blepharitis" refers to an eye condition, characterised by chronic inflammation of the eyelid. Blepharitis may typically develop as a result of a bacterial infection of the eyelids, typically with a *Staphylococcus* species, mite infestation, a skin condition such as seborrhoeic dermatitis or skin rosacea, meibomian gland dysfunction or a combination thereof.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integers or steps. Thus, the use of the term "comprising" and the like indicates that the listed integers are required or mandatory, but that other integers are optional and may or may not be present. By "consisting of" is meant including, and limited to, whatever follows the phrase "consisting of". Thus, the phrase "consisting of" indicates that the listed elements are required or mandatory, and that no other elements may be present. By "consisting essentially of" is meant including any elements listed after the phrase, and limited to other elements that do not interfere with or contribute to the activity or action specified for the listed elements. Thus, the phrase "consisting essentially of" indicates that the listed elements are required or mandatory, but that other elements are optional and may or may not be present depending upon whether or not they affect the activity or action of the listed elements.

By "derivative" is meant a molecule, such as a small molecule, that has been derived from the basic molecule by modification, for example by conjugation or complexing with other chemical moieties or by modification techniques as would be understood in the art, such as removal of an oxygen or hydrogen atom, and/or addition of an oxygen atom or a sulfo or phospho group. The term "derivative" also includes within its scope alterations that have been made to a parent molecule including additions or deletions (e.g. removal of an oxygen or hydrogen atom, and/or addition of an oxygen atom or a sulfo or phospho group) that provide for functionally equivalent molecules.

As used herein, the term "dosage unit form" refers to physically discrete units suited as unitary dosages for the subject to be treated, each unit containing a predetermined quantity of active material calculated to produce the desired therapeutic effect in association with the required pharmaceutically acceptable vehicle.

The phrase "dry eye syndrome" is used herein to refer to an ocular disorder of the normal tear film, resulting from decreased tear production, excessive tear evaporation or an abnormality in the production of mucus or lipids normally found in the tear layer. Dry eye syndrome may occur as a result of age; hormonal changes; autoimmune diseases, such as primary Sjögren's syndrome, rheumatoid arthritis, Stevens-Johnson syndrome, cicatricial pemphigoid or lupus erythematosus; medication use, such as antihistamines, antidepressants, beta-blockers, oral isotretinoin and oral contraceptives; lifestyle factors resulting in decreased blinking, such as tasks which require close visual attention; certain conditions resulting in an impaired ability to blink such as stroke or Bell's palsy; chemical burns to the eye; meibomian gland dysfunction; meibomian gland disease; rosacea; an infection, such as blepharitis; or trauma, such as surgery.

By "effective amount," in the context of treating or inhibiting the development of a condition is meant the administration of an amount of an agent or composition to an individual in need of such treatment or prophylaxis, either in a single dose or as part of a series, that is effective for the prevention of incurring a symptom, holding in check such symptoms, and/or treating existing symptoms, of that condition. The effective amount will vary depending upon the health and physical condition of the individual to be treated, the taxonomic group of individual to be treated, the formulation of the composition, the assessment of the medical situation, and other relevant factors. It is expected that the amount will fall in a relatively broad range that can be determined through routine trials.

The phrase "honey analogue" is used herein to refer to a sugar syrup solution that mimics the chemical and/or physical properties of natural, raw honey. A honey analogue may comprise, for example, glucose, fructose, glycerol, water and hydrogen peroxide and/or glucose peroxidase enzyme.

As used herein, the phrase "inhibit the development of" refers to a prophylactic treatment which increases the resistance of a subject to developing the disease, disorder or condition or, in other words, decreases the likelihood that the subject will develop the disease, disorder or condition as well as a treatment after the disease, disorder or condition has begun in order to reduce or eliminate it altogether or prevent it from becoming worse. This phrase also includes within its scope preventing the disease, disorder or condition from occurring in a subject which may be predisposed to the disease, disorder or condition but has not yet been diagnosed as having it.

The phrase *"Leptospermum* species" refers to a genus of plants in the Myrtle family (Myrtaceae). This family includes, but is not limited to, the species *Leptospermum scoparium, Leptospermum polygalifolium, Leptospermum semibaccatum, Leptospermum trinervium, Leptospermum whitei, Leptospermum speciosum, Leptospermum petersonii* and *Leptospermum liversidgei.*

By "modulating" is meant increasing or decreasing, either directly or indirectly, the level or functional activity of a target molecule or response, such as an inflammatory response. For example, an agent may indirectly modulate the level/activity by interacting with a molecule other than the target molecule.

By "pharmaceutically acceptable carrier" is meant a pharmaceutical vehicle comprised of a material that is not biologically or otherwise undesirable, i.e., the material may be administered to a subject along with the selected active agent without causing any or a substantial adverse reaction. Carriers may include excipients and other additives such as diluents, fillers, detergents, colouring agents, wetting or emulsifying agents, preservatives and the like.

Similarly, a "pharmaceutically acceptable" salt or derivative of a compound as provided herein is a salt or derivative that this not biologically or otherwise undesirable.

The terms "reduce", "inhibit" and grammatical equivalents when used in reference to the level of a substance and/or phenomenon in a first sample relative to a second sample, mean that the quantity of substance and/or phenomenon in the first sample is lower than in the second sample by any amount that is statistically significant using any art-accepted statistical method of analysis. When these terms are used to refer to the action of a compound (e.g. gluconic acid) or agent, the first sample may be a sample in the presence of the compound or agent and the second sample may be a comparative sample without the compound or agent. In one embodiment, the reduction may be determined subjectively, for example when a patient refers to their subjective perception of disease symptoms, such as pain, itchiness, etc. or increased stinging upon administration of a composition. In another embodiment, the reduction may be determined objectively, for example when the pH of a composition is lower in one sample compared to a control sample. In another embodiment, the quantity of substance and/or phenomenon in the first sample is at least 10% lower than the quantity of the same substance and/or phenomenon in a second sample. In another embodiment, the quantity of the substance and/or phenomenon in the first sample is at least 25% lower than the quantity of the same substance and/or phenomenon in a second sample. In yet another embodiment, the quantity of the substance and/or phenomenon in the first sample is at least 50% lower than the quantity of the same substance and/or phenomenon in a second sample. In a further embodiment, the quantity of the substance and/or phenomenon in the first sample is at least 75% lower than the quantity of the same substance and/or phenomenon in a second sample. In yet another embodiment, the quantity of the substance and/or phenomenon in the first sample is at least 90% lower than the quantity of the same substance and/or phenomenon in a second sample.

As used herein, the phrase "respiratory condition" refers to any disease, condition or disorder of the respiratory tract, wherein the respiratory tract includes the nose, nasal passages, sinuses, throat, pharynx, voice box, larynx, trachea, bronchi, bronchioles and lungs.

As used herein, the term "salts" include any pharmaceutically acceptable salt which, upon administration to the recipient, is capable of providing (directly or indirectly) a desired compound (e.g. gluconic acid or gluconate), or an active metabolite or residue thereof. The term "pharmaceutically acceptable salts" refers without limitation to derivatives of the disclosed compounds wherein the parent compound is modified by converting an existing acid or base moiety to its salt form (e.g. by reacting the free base group with a suitable organic acid). Examples of pharmaceutically acceptable salts include, but are not limited to, mineral or organic acid salts of basic residues such as amines; alkali or organic salts of acidic residues such as carboxylic acids; and the like. Representative acid addition salts include acetate, adipate, alginate, ascorbate, aspartate, benzenesulfonate, benzoate, bisulfate, borate, butyrate, camphorate, camphorsulfonate, citrate, cyclopentanepropionate, digluconate, dodecylsulfate, ethanesulfonate, fumarate, glucoheptonate, glycerophosphate, hemisulfate, heptonate, hexanoate, hydrobromide, hydrochloride, hydroiodide, 2-hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, toluenesulfonate, undecanoate and valerate salts, and the like. Representative alkali or alkaline earth metal salts include sodium, lithium, potassium, calcium, magnesium, and the like, as well as nontoxic ammonium, quaternary ammonium, and amine cations, including, but not limited to ammonium, tetramethylammonium, tetraethylammonium, methylamine, dimethylamine, trimethylamine, triethylamine, ethylamine, and the like. The salt may also include a zinc, copper or ferrous salt. The pharmaceutically acceptable salts of the present invention include the conventional nontoxic salts of the parent compound formed, for example, from non-toxic inorganic or organic acids. The pharmaceutically acceptable salts can be synthesised from the parent compound which contains a basic or acidic moiety by conventional chemical methods. Generally, such salts can be prepared by reacting the free acid or base forms of these compounds with a stoichiometric amount of the appropriate base or acid in water or in an organic solvent, or in a mixture of the two; generally, nonaqueous media like ether, ethyl acetate, ethanol, isopropanol, or acetonitrile are preferred. Lists of suitable salts are found in, for example, Remington (1985) Remington's Pharmaceutical Sciences, Mack Publishing Co., Easton, Pa., 17th edition; Stahl and Wermuth (2002) Pharmaceutical Salts: Properties, Selection, and Use, Wiley-VCH; and Berge et al. (1977) Journal of Pharmaceutical Science, 66: 1-19, each of which is incorporated herein by reference in its entirety.

The terms "stabilise" and "stabilising" as used herein refer to inhibiting or minimising a change in a phenomenon or value, such as the pH of a composition. For example, stabilising the pH refers to substantially inhibiting or minimising the change in the pH over a period of time. In particular embodiments, stabilising means that the pH value changes by no more than about 0.5, 0.4, 0.3, 0.2 or 0.1 over a desired time period, such as one month, two months, three months, four months, five months, six months, one year, two years or three years.

The term "subject" as used herein refers to a vertebrate subject, particularly a mammalian subject, for whom therapy or prophylaxis is desired. Suitable subjects include, but are not limited to, primates; avians (birds); livestock animals such as sheep, cows, horses, deer, donkeys and pigs; laboratory test animals such as rabbits, mice, rats, guinea pigs and hamsters; companion animals such as cats and dogs; and captive wild animals such as foxes, deer and dingoes. In particular, the subject is a human. However, it will be understood that the aforementioned terms do not imply that symptoms are present.

As used herein, the phrase "substantially sourced from *Leptospermum* species" refers to honey that is produced from nectar, at least 50%, especially at least 75%, and more especially at least 85%, 95% or 98% of which is sourced from one or more *Leptospermum* species.

As used herein, the terms "treatment", "treating", and the like, refer to obtaining a desired pharmacologic and/or physiologic effect. The effect may be therapeutic in terms of a partial or complete cure for a disease, disorder or condition and/or adverse effect attributable to the disease, disorder or condition. These terms also cover any treatment of a condition, disorder or disease in a subject, particularly in a human, and include: (a) inhibiting the disease, disorder or condition, i.e. arresting its development; or (b) relieving the disease, disorder or condition, i.e. causing regression of the disease, disorder or condition. These terms also encompass relieving the symptoms of a disease, disorder or condition.

Each embodiment described herein is to be applied *mutatis mutandis* to each and every embodiment unless specifically stated otherwise.

### 2. Compositions

The present invention is based on the discovery that gluconic acid or a pharmaceutically acceptable salt or derivative thereof can be added to compositions comprising honey or a honey analogue and a pharmaceutically acceptable carrier to stabilise the pH of the composition. Such compositions may be used for treating or inhibiting the development of an infection or an inflammatory condition.

Accordingly, in one aspect, there is provided a composition comprising honey or a honey analogue, gluconic acid or a pharmaceutically acceptable salt or derivative thereof and a pharmaceutically acceptable carrier.

The amount of honey or honey analogue in the composition will depend on the intended use of the composition, including the site of application, the route of administration and/or the disorder being treated or prevented. In some embodiments, the honey or honey analogue is in an amount in the range of from about 2% to about 98% w/w (and all one tenth integer percentages in between), about 2% to about 90% w/w, about 2% to about 80% w/w, about 2% to about 70% w/w, about 2% to about 60% w/w, about 2% to about 50% w/w, about 2% to about 45% w/w, about 2% to about 40% w/w, about 2% to about 35% w/w, about 2% to about 30% w/w, about 2% to about 25% w/w, about 2% to less than 25% w/w, about 2% to about 20% w/w, about 5% to about 19% w/w, about 7% to about 18% w/w, about 10% to about 18% w/w, about 14 to about 17% w/w or about 15% to about 17% w/w. In particular embodiments, the honey or honey analogue is in an amount of about 16.5% w/w.

In particular embodiments, the composition contains honey. The honey used in the composition is natural, raw honey sourced from any plant species or a combination of honey sourced from multiple plant species. In specific embodiments, such as when high antimicrobial activity is desired, the honey may be substantially sourced from *Leptospermum* species, which is bacteriostatic at low concentrations. For example, the honey may be substantially sourced from *Leptospermum* species selected from the group consisting of *Leptospermum scoparium, Leptospermum polygalifolium, Leptospermum semibaccatum, Leptospermum trinervium, Leptospermum whitei, Leptospermum speciosum, Leptospermum liversidgei* and a combination thereof.

The gluconic acid or a pharmaceutically acceptable salt or derivative thereof may be present in an amount sufficient to maintain the pH within a desired range for a suitable period of time, such as at least six months, one year, two years or three years. In some embodiments, the gluconic acid or a pharmaceutically acceptable salt or derivative thereof is in an amount in the range of from about 0.05% to about 10% w/w (and all one tenth integer percentages in between), about 0.05% to about 8%, about 0.1% to about 5% w/w, about 0.1% to about 3% w/w, about 0.1% to about 2% w/w, about 0.1% to about 1% w/w, about 0.1% to about 0.7% w/w or about 0.1% to about 0.5% w/w. In particular embodiments, the gluconic acid or a pharmaceutically acceptable salt or derivative thereof is in an amount of about 0.1%, 0.2%, 0.3% 0.4%, 0.5%, 0.6%, 0.7%, 0.8%, 0.9% or 1% w/w; especially about 0.3% w/w. Natural, raw honey is known to contain a number of acidic components, including gluconic acid. The amount of gluconic acid or a pharmaceutically acceptable salt or derivative thereof recited herein does not include the gluconic acid content in the raw honey or honey analogue itself, i.e. it is the amount of gluconic acid or a pharmaceutically acceptable salt or derivative thereof which is added to the honey or honey analogue and carrier.

In some embodiments, the composition comprises gluconic acid. In particular embodiments, the gluconic acid is D-gluconic acid.

In alternative embodiments, the composition comprises a pharmaceutically acceptable salt of gluconic acid, representative examples of which include sodium gluconate, calcium gluconate, potassium gluconate, magnesium gluconate, zinc gluconate, copper gluconate and ferrous gluconate. In particular embodiments, the pharmaceutically acceptable salt of gluconic acid is selected from the group consisting of sodium gluconate, calcium gluconate, potassium gluconate, magnesium gluconate and zinc gluconate; especially sodium gluconate, calcium gluconate, potassium gluconate and magnesium gluconate. In specific embodiments, the pharmaceutically acceptable salt of gluconic acid is sodium gluconate. In particular embodiments, the gluconate salt is in the D configuration, i.e. is D-gluconate.

Also contemplated is the use of a derivative of gluconic acid in the composition of the invention. For example, suitable gluconic acid derivatives include but are not limited to, 2-dehydro-3-deoxy-6-phospho-D-gluconic acid, 6-phospho-5-dehydro-2-deoxy-D-gluconic acid, 2-dehydro-3,6-dideoxy-6-sulfo-D-gluconic acid, 2-deoxy-D-gluconic acid, 6-phospho-D-gluconic acid, 3-dehydro-2-deoxy-D-gluconic acid, 5-dehydro-D-gluconic acid, 5-dehydro-L-gluconic acid, 2-dehydro-3-deoxy-D-gluconic acid, 6-phospho-2-dehydro-D-gluconic acid, 2-amino-2-deoxy-D-gluconic acid, 2-dehydro-D-gluconic acid, 6-deoxy-6-sulfo-D-gluconic acid, 3-dehydro-D-gluconic acid, 5-dehydro-2-deoxy-D-gluconic acid, 3-dehydro-2-deoxy-D-gluconic acid, 2,5-didehydro-D-gluconic acid, 2-keto-L-gluconic acid and pharmaceutically acceptable salts and combinations thereof. For example, in some embodiments, the gluconic acid derivative is a 6-phosphonatooxy-D-gluconate, 6-deoxy-6-sulfo-D-gluconate, 2-deoxy-D-gluconate, 2-dehydro-D-gluconate, 3-dehydro-D-gluconate, 6-phospho-D-gluconate, 5-dehydro-2-deoxy-D-gluconate, 3-dehydro-2-deoxy-D-gluconate, 2-dehydro-3-deoxy-D-gluconate, 2-amino-2-deoxy-D-gluconate, 5-dehydro-D-gluconate, 5-dehydro-L-gluconate, 2,5-didehydro-D-gluconate, 2-dehydro-3-deoxy-6-phosphonato-D-gluconate, 6-phosphonato-5-dehydro-2-deoxy-D-gluconate, 6-phospho-2-dehydro-D-gluconate, 2-dehydro-3,6-dideoxy-6-sulfo-D-gluconate or 2-keto-L-gluconate salt, such as a sodium, potassium, calcium, magnesium, zinc, copper or ferrous salt of any one of the foregoing.

The pharmaceutically acceptable carrier will depend on the route of administration and the form of the composition. In preferred embodiments, the carrier is a liquid carrier. In such embodiments, the pharmaceutically acceptable carrier is an aqueous carrier, glycerol or a combination thereof. Suitable aqueous carriers include, but are not limited to, water, saline and an aqueous solution comprising water and a miscible solvent such as glycerol; especially water or saline.

When saline is used as a carrier, the saline is preferably isotonic for the point of administration, such as the eye, nasal cavity or respiratory tract. For example, in some embodiments the saline comprises about 0.15% to about 8% (and all one tenth integer percentages in between), about 0.18% to about 7%, about 0.22% to about 5%, about 0.45% to about 3%, about 0.5% to about 2% or about 0.65% to about 1.5% w/v sodium chloride; especially about 0.9% w/v sodium chloride.

In some embodiments where the aqueous carrier is not isotonic, for example water, the aqueous carrier may contain one or more tonicity agents. Suitable tonicity agents include, but are not limited to, boric acid, sodium acid phosphate buffer, sodium chloride, glucose, potassium chloride, calcium chloride, magnesium chloride, polypropylene glycol, glycerol or salts or combinations thereof. The tonicity agent may be present in the composition in an amount that provides isotonicity with the point of administration, such as the eye or nasal cavity, for example in the range of from about 0.02 to about 15% w/w (and all one tenth integer percentages in between).

Alternatively, the composition may be hyper-isotonic, either by the amount of honey or honey analogue in the composition or the addition of increased amounts of tonicity agent.

In some embodiments, the carrier is glycerol. In such embodiments, the glycerol is present in an amount in the range of from about 0.1% to about 60% w/w (and all one tenth integer percentages in between). For example, in come embodiments, the glycerol is present in an amount in the range of from about 0.1 to about 55%, about 0.1 to about 50%, about 0.1 to about 45%, about 0.1 to about 40%, about 0.1 to about 35%, about 0.1 to about 30%, about 0.1 to about 25%, about 0.1 to about 20%, about 0.1 to about 15%, about 0.1 to about 10%, about 0.1 to about 5%, about 0.1 to about 1% w/w; especially about 0.1, 0.2, 0.3, 0.4, 0.5, 0.6, 0.7, 0.8, 0.9 or 1% w/w; most especially about 0.2% w/w. In some embodiments, the glycerol is present in an amount in the range of from about 40% to about 60%, about 45% to about 55% or about 45, 46, 47, 48, 49, 50, 51, 52, 53, 54 or 55% w/w, especially about 50% w/w. While glycerol may be added to the composition in substantially pure form, in some embodiments, glycerol is added to the composition in the form of glycerine, which is a commercially available form of glycerol and often contains at least about 95% glycerol.

While preservative free compositions are contemplated, in particular embodiments, such as when the amount of honey or honey analogue in the composition is less than about 50% w/w and/or when the composition is in the form of a multiple dose unit, the composition further comprises a preservative. Suitable preservatives include, but are not limited to, benzoic acid, a flavonoid (e.g. flavonols, flavones, flavanones, isoflavones, anthocyanidins or a combination thereof, especially, myricetin, tricetin, quercetin, luteolin, kaempferol, kaempferol 8-methyl ether, pinocembrin or chrysin), a phenolic acid (e.g. gallic acid, ellagic acid, chlorogenic acid, caffeic acid, p-coumaric acid, ferulic acid, syringic acid or a combination thereof), abscisic acid, sorbic acid, sodium perborate, stabilised oxychloro complex, polyquaternium-1, phenylmercuric acid, benzalkonium chloride, chlorobutanol, phenylmercuric acetate, phenylmercuric nitrate, chlorhexidine acetate, benzododecinium bromide, cetrimonium chloride, thiomersal, methyl parahydroxybenzoate, propyl parahydroxybenzoate, polyquaternium ammonium chloride, polyaminopropyl biguanide, hydrogen peroxide, ascorbic acid or a pharmaceutically acceptable salt or combination thereof. In particular embodiments, the preservative is selected from the group consisting of benzoic acid, a flavonoid, a phenolic acid, abscisic acid, sorbic acid, ascorbic acid and pharmaceutically acceptable salts and combinations thereof; especially benzoic acid or a pharmaceutically acceptable salt thereof, such as sodium benzoate.

The preservative may be present in the composition in an amount that provides adequate preservative activity. For example, the preservative may be present in an amount in the range of from about 0.001 to about 0.7% (and all one hundredth integer percentages in between), about 0.002% to about 0.6%, about 0.003% to about 0.5%, about 0.004% to about 0.5%, about 0.005% to about 0.5%, about 0.01% to about 0.4%, about 0.05% to about 0.4%, about 0.05% to about 0.3%, or about 0.05% to about 0.25% w/w; especially about 0.05% to about 0.25% w/w.

Honey contains several compounds that have antioxidant or antimicrobial activity and, therefore, act as natural preservatives. In particular embodiments, the composition of the invention includes the addition of one or more of the above preservative compounds to supplement natural preservatives found in the honey or honey analogue but in sub-effective amounts after dilution of the honey or honey analogue. For example, in particular embodiments, the preservative may include, but is not limited to, abscisic acid, sodium benzoate, a flavonoid, benzoic acid, a phenolic acid or a salt or combination thereof. This may avoid the tendency of the subjects to develop acute sensitivity to preservatives, particularly those that are commonly used in ophthalmic compositions. In some embodiments, the preservative acts together with natural honey or honey analogue preservatives. Suitable amounts of the preservative that are to be added to the composition are as discussed *supra.* Such amounts do not include the preservative content in the raw honey or honey analogue itself, i.e. it is the amount of preservative which is added to the raw honey or honey analogue and carrier.

The pH of the composition will depend on the intended use and route of administration of the composition. In suitable embodiments, the pH of the composition is in the range of from about 3 to about 5 (and all one tenth integers in between), about 3.5 to about 5, about 3.5 to about 4.5, about 3.8 to about 4.2, about 3.9 to about 4.3, or about 3.9 to about 4.1. In particular embodiments, the pH of the composition is about 3.5, 3.6, 3.7, 3.8, 3.9, 4, 4.1, 4.2, 4.3, 4.4 or 4.5. In some embodiments, the composition has a pH in any one of the ranges disclosed above for at least one month, two months, four months, six months, one year, two years or three years.

The composition may further comprise a rheology modifier, for example, to alter the surface tension and flow of the composition, especially when the composition comprises a minimal amount of honey or honey analogue. Suitable rheology modifiers include, but are not limited to, a hydrocolloid, gum arabic, xanthan gum, guar gum, locust bean gum, carboxymethylcellulose, alginate, starch (e.g. from rice, corn, potato or wheat), carrageenan, konjac, aloe vera gel, agarose, pectin, tragacanth, curdlan gum, gellan gum, scleroglucan, hyaluronic acid, chitosan-polyvinyl alcohol or derivatives thereof, polyvinyl alcohol, polyethylene glycol, polyvinyl pyrrolidone, Carbopol or derivatives thereof, dextran, methylcellulose, hydroxypropylmethylcellulose, hydroxyethylcellulose, hydroxypropylcellulose, hydroxypropyl guar or a combination thereof. In particular embodiments, the rheology modifier is selected from the group consisting of gum arabic, xanthan gum, guar gum, locust bean gum, carboxymethylcellulose, alginate, starch and combinations thereof; especially gum arabic.

Also contemplated is the addition of one or more polysaccharides to the composition that may have other useful therapeutic properties but may not be classified as rheology modifiers. For example, in some embodiments, the composition further comprises a polysaccharide selected from the group consisting of chitosans, chitins, dermatans, hyaluronates, heparans, dermatans, chondroitins, heparins and combinations thereof.

It may be useful to add a surfactant or wetting agent to the composition, for example, to emulsify an aqueous carrier with an oil when the composition comprises an oil, and/or to improve cleaning or mobility of fatty acids (e.g. from the meibomian glands). Accordingly, in some embodiments, the composition further comprises a surfactant or wetting agent. Suitable surfactants and wetting agents include, but are not limited to, benzalkonium chloride, Cetomacrogol 1000, polysorbate, dioctyl sodium sulphosuccinate, fatty alcohol ethoxylates, alkylphenol polyethylene glycols, alkyl mercaptan polyethylene glycols, fatty amine ethoxylates, fatty acid ethoxylates, polypropylene glycol ethoxylates, fatty acid alkylolamides, alkyl polyglycosides, N-alkylpolyhydroxy fatty acid amide, N-alkoxypolyhydroxy fatty acid amide, sucrose esters, sorbitol esters, esters of sorbitol polyglycol ethers or combinations thereof. The amount of surfactant or wetting agent added to the composition will depend on the intended role of the surfactant or wetting agent and the nature and amount of the other components of the composition. For example, the surfactant or wetting agent may be present in an amount in the range of from about 0.5% to about 7% w/w (and all one tenth integer percentages in between), especially about 1% to about 5% w/w.

As described above, the composition may also further comprise one or more oils, such as almond oil including sweet almond oil, castor oil, mineral oil, citrus oil, clove oil, tea tree oil, olive oil, peanut oil, coconut oil, soybean oil, lavender oil, garlic oil or seed oils such as canola oil, cottonseed oil, linseed oil, grapeseed oil, safflower oil, sesame oil or sunflower oil; especially almond oil, particularly sweet almond oil. Such oils may be included in the composition in the form of an oil-in-water emulsion, optionally with a surfactant, with an aqueous carrier. The oil may be present in an amount in the range of from, for example, about 0.2% to about 20% w/w (and all one tenth integer percentages in between).

The composition may also further comprise a chelating agent. Suitable chelating agents include, but are not limited to, amino carboxylic acids and salts thereof such as ethylenediamine-tetraacetic acid (EDTA), nitrilotriacetic acid, nitrilotripropionic acid, diethylenetriamine-pentacetic acid, 2-hydroxyethyl-ethylenediamine-triacetic acid, 1,6-diamino-hexamethylene-tetraacetic acid, 1,2-diamino-cyclohexane tetraacetic acid, O,O'-bis(2-aminoethyl)-ethyleneglycol-tetraacetic acid, 1,3-diaminopropane-tetraacetic acid, N,N-bis(2-hydroxybenzyl)ethylenediamine-N,N-diacetic acid, ethylenediamine-N,N'-diacetic acid, ethylenediamine-N,N'-dipropionic acid, triethylenetetramine-hexaacetic acid, 7,19,30-trioxa-1,4,10,13,16,22,27,33-octaazabicyclo[11,11,11]pentatriacontane (O-bis-tren), ethylenediamine-N,N'-bis(methylenephosphonic acid), iminodiacetic acid, N,N-bis(2-hydroxyethyl)glycine (DHEG), 1,3-diamino-2-hydroxypropane-tetraacetic acid, 1,2-diaminopropane-tetraacetic acid, ethylenediamine-tetrakis(methylenephosphonic acid) or N-(2-hydroxyethyl)iminodiacetic acid or a pharmaceutically acceptable salt thereof; especially a pharmaceutically acceptable salt or mixed salt of EDTA, such as disodium, trisodium, tetrasodium, dipotassium, tripotassium, lithium, dilithium, ammonium, diammonium, calcium or calcium-disodium; more especially a disodium or tetrasodium salt of EDTA. In particular embodiments, the chelating agent is disodium EDTA.

The composition may also further comprise one or more alcohols, such as isopropanol, benzyl alcohol, cetearyl alcohol or ethanol. The alcohol may be present in the composition in an amount in the range of from about 0.2% to about 12% w/w (and all one tenth integer percentages in between).

For particular uses and routes of administration, such as topical ocular administration, the composition further comprises a lubricant, especially an ocular lubricant. Suitable ocular lubricants include, but are not limited to, glucose, glycerol, polyethylene glycol or propylene glycol, especially glycerol. The lubricant may be present, for example, in an amount in the range of from about 0.2% to about 20% w/w (and all one tenth integer percentages in between).

In some embodiments, the composition further comprises a cyclodextrin. In such embodiments, one or more of the honey components may form a complex with the cyclodextrin. Suitable cyclodextrins include, but are not limited to, o-cyclodextrin, β-cyclodextrin, γ-cyclodextrin, 2-hydroxypropyl-β-cyclodextrin, sulfobutylether β-cyclodextrin sodium salt, methylated β-cyclodextrin and 2-hydroxypropyl-γ-cyclodextrin; especially α-cyclodextrin, β-cydodextrin or γ-cyclodextrin; most especially α-cyclodextrin.

The composition may further comprise other natural remedies or plant extracts. For example, the composition may further comprise herbs or fungi or extracts thereof, such as those used in traditional herbology, including Chinese herbology, Islamic herbology and Indian herbology; or plant and/or flower extracts, such as lavender, jasmine, violet, rose, marigold, chamomile, frangipani, cactus flower, aloe vera, jojoba, rosehip, pomegranate, green tea, lemongrass, mint, linden flower, *Glycyrrhiza uralensis, Panax ginseng, Arnica montana* or honeysuckle flower extract or a combination thereof.

In particular embodiments, the presence of an antioxidant may be desired. Suitable antioxidants include, but are not limited to, vitamin E (tocopherol), vitamin C (ascorbic acid), ubiquinone, idebenone, lycopene, resveratrol, niacinamide or derivatives thereof. The antioxidant may be present, for example, in an amount in the range of from about 0.1% to about 4% w/w (and all one tenth integer percentages in between).

While the composition may not comprise further active agents, the administration of other active agents concurrently with the composition or the inclusion of other active agents in the composition is within the scope of the invention. For example, in some embodiments, the composition further comprises or may be administered concurrently with one or more anti-inflammatory agents, immunosuppressants, antibiotics, anti-virals, anti-protozoals, anti-fungals or anthelmintics. The composition may be therapeutically used after the other active agent or may be therapeutically used together with the other active agent. The composition may be administered separately, simultaneously or sequentially with the other active agent.

Accordingly, in some embodiments, the composition further comprises an anti-inflammatory agent, immunosuppressant, antibiotic, anti-viral, anti-protozoal, anti-fungal or anthelmintic.

Suitable immunosuppressants include prednisone, methylprednisolone, dexamethasone, betnesol, beclometasone, betamethasone, fluticasone, flunisolide, mometasone, triamcinolone, fluocinolone, hydrocortisone, budesonide, prednisolone, tofacitinib, cyclosporine, cyclophosphamide, nitrosoureas, platinum compounds, methotrexate, azathioprine, mercaptopurine, fluorouracil, dactinomycin, anthracyclines, mitomycin C, bleomycin, mithramycin, antithymocyte globulin, thymoglobulin, muromonab-CD3, basiliximab, daclizumab, tacrolimus, sirolimus, everolimus, infliximab, etanercept, IFN-β, mycophenolic acid or mycophenolate, fingolimod, azathioprine, leflunomide, abatacept, adalimumab, anakinra, certolizumab, golimumab, ixekizumab, natalizumab, rituximab, secukinumab, toclizumab, ustekinumab, vedolizumab, glatiramer acetate, dimethyl fumarate, diroximel fumarate, teriflunomide, siponimod, cladribine, ocrelizumab, natalizumab, alemtuzumab and myriocin.

Exemplary anti-inflammatory agents include NSAIDs (e.g. acetylsalicylic acid (aspirin), diclofenac, diflusinal, etodolac, fenbufen, fenoprofen, flufenisal, flurbiprofen, ibuprofen, indomethacin, ketoprofen, ketorolac, meclofenamic acid, mefenamic acid, meloxicam, nabumetone, naproxen, nimesulide, nitroflurbiprofen, olsalazine, oxaprozin, phenylbutazone, piroxicam, sulfasalazine, sulindac, tolmetin, zomepirac, celecoxib, deracoxib, etoricoxib, mavacoxib, phenazone, suprofen or parecoxib), disease-modifying antirheumatic drugs (DMARDs) (e.g. methotrexate, leflunomide, sulfasalazine, hydroxychloroquinone, penicillamine, anatacept, baricitinib, cetolizumab, sarilumab, tocilizumab or tofacitinib), prednisone, methylprednisolone, dexamethasone, hydrocortisone, budesonide, prednisolone, etanercept, golimumab, infliximab, adalimumab, anakinra, rituximab, natalizumab and abatacept.

Exemplary antibiotics include, but are not limited to, quinolones (e.g. amifloxacin, cinoxacin, ciprofloxacin, enoxacin, fleroxacin, flumequine, lomefloxacin, nalidixic acid, norfloxacin, ofloxacin, levofloxacin, lomefloxacin, oxolinic acid, pefloxacin, rosoxacin, temafloxacin, tosufloxacin, sparfloxacin, clinafloxacin, gatifloxacin, moxifloxacin, gemifloxacin, or garenoxacin), tetracyclines, glycylcyclines or oxazolidinones (e.g. chlortetracycline, demeclocycline, doxycycline, lymecycline, methacycline, minocycline, oxytetracycline, tetracycline, tigecycline, linezolide or eperezolid), aminoglycosides (e.g. amikacin, arbekacin, butirosin, dibekacin, fortimicins, gentamicin, neomycin, kanamycin, menomycin, netilmicin, ribostamycin, sisomicin, spectinomycin, streptomycin, framycetin or tobramycin), β-lactams (e.g. imipenem, meropenem, biapenem, cefaclor, cefadroxil, cefamandole, cefatrizine, cefazedone, cefazolin, cefixime, cefmenoxime, cefodizime, cefonicid, cefoperazone, ceforanide, cefotaxime, cefotiam, cefpimizole, cefpiramide, cefpodoxime, cefsulodin, ceftazidime, cefteram, ceftezole, ceftibuten, ceftizoxime, ceftriaxone, cefuroxime, cefuzonam, cephacetrile, cephalexin, cephaloglycin, cephaloridine, cephalothin, cephapirin, cephradine, cefinetazole, cefoxitin, cefotetan, azthreonam, carumonam, flomoxef, moxalactam, amdinocillin, amoxicillin, ampicillin, azlocillin, carbenicillin, benzylpenicillin, carfecillin, cloxacillin, dicloxacillin, methicillin, mezlocillin, nafcillin, oxacillin, penicillin G, piperacillin, sulbenicillin, temocillin, ticarcillin, cefditoren, cefdinir, ceftibuten or cefozopran), rifamycins, macrolides (e.g. azithromycin, clarithromycin, erythromycin, oleandomycin, rokitamycin, rosaramicin, roxithromycin or troleandomycin), ketolides (e.g. telithromycin or cethromycin), coumermycins, lincosamides (e.g. clindamycin or lincomycin), polymyxins (e.g. colistin), dibrompropamidine, sulfacetamide, fusidic acid, gramicidin, chloramphenicol, methylglyoxal or dihydroxyacetone.

Suitable anti-virals include, but are not limited to, abacavir sulfate, acyclovir sodium, amantadine hydrochloride, amprenavir, cidofovir, delavirdine mesylate, didanosine, efavirenz, famciclovir, fomivirsen sodium, foscarnet sodium, ganciclovir, indinavir sulfate, lamivudine, lamivudine/zidovudine, nelfinavir mesylate, nevirapine, oseltamivir phosphate, ribavirin, rimantadine hydrochloride, ritonavir, saquinavir, saquinavir mesylate, stavudine, valacyclovir hydrochloride, zalcitabine, zanamivir or zidovudine.

Exemplary anti-protozoals include atovaquone, chloroquine hydrochloride, chloroquine phosphate, doxycycline, hydroxychloroquine sulfate, mefloquine hydrochloride, primaquine phosphate, pyrimethamine, metronidazole, metronidazole hydrochloride and pentamidine isethionate.

Illustrative anti-fungals include, but are not limited to, amphotericin B, amphotericin B cholesteryl sulfate complex, amphotericin B lipid complex, amphotericin B liposomal, anidulafungin, caspofungin, clotrimazole, fluconazole, flucytosine, griseofulvin, griseofulvin microsize, griseofulvin ultramicrosize, isavuconazonium, itraconazole, ketoconazole, micafungin, miconazole, nystatin, posaconazole, terbinafine, voriconazole, fosfluconazole, isavuconazole, candicidin, hamycin, natamycin, bifonazole, butoconazole, econazole, fenticonazole, isoconazole, luliconazole, omoconazole, oxiconazole, sertaconazole, sulconazole, tioconazole, albaconazole, efinaconazole, terconazole, abafungin, amofolfin, butenafine, naftifine, ciclopirox, tolnafate, orotomide, oteseconazole (VT-1161, (2R)-2-(2,4-difluorophenyl)-1,1-difluoro-3-(tetrazol-1-yl)-1-[5-[4-(2,2,2-trifluoroethoxy)phenyl]pyridin-2-yl]propan-2-ol), ibrexafungerp (SCY-078, 2-(2-amino-2,3,3-trimethylbutoxy)-1,6a,8,10a-tetramethyl-8-(3-methylbutan-2-yl)-3-(5-(pyridin-4-yl)-1H-1,2,4-triazol-1-yl)-1,3,4,6,6a,7,8,9,10,10a,10b,11,12,12a-tetradecahydro-2H-1,4a-(methanooxymethano)chrysene-7-carboxylic acid), rezafungin (CD-101, 2-(((2R,9S,11R,12R,14aS,15S,16S,20S,25aS)-23-((1S,2S)-1,2-dihydroxy-2-(4-hydroxyphenyl)ethyl)-2,11,15-trihydroxy-6,20-bis((R)-1-hydroxyethyl)-16-methyl-5,8,14,19,22,25-hexaoxo-9-(4"-(pentyloxy)-[1,1':4',1"-terphenyl]-4-carboxamido)tetracosahydro-1H-dipyrrolo[2,1-c:2',1'-l][1,4,7,10,13,16]hexaazacyclohenicosin-12-yl)oxy)-N,N,N-trimethylethan-1-aminium acetate), and salts and combinations thereof.

Anthelmintics contemplated include, but are not limited to, a benzimidazole (e.g. albendazole, mebendazole, thiabendazole, fenbendazole, triclabendazole and flubendazole), abamectin, ivermectin, diethylcarbamazine, pyrantel pamoate, levamisole, salicylanilide, niclosamide, oxyclozanide, rafoxanide, nitazoxanide, oxamniquine, praziquantel, an octadepsipeptide (e.g. emodepside), monepantel, a spiroindole (e.g. derquantel), antemisinin, piperazine, morantel, pyrantel, tribendimidine, and salts and combinations thereof.

Further active agents which may also be added to the composition or concurrently administered with the composition, include, but are not limited to, other antimicrobial agents, such as sorbic acid, chlorhexidine, polyhexamethylene biguanide or chlorobutanol; an anti-allergy agent such as an antihistamine or a mast cell stabiliser; a vasoconstrictor such as naphazoline, phenylephrine, oxymetazoline or tetrahydrozoline; a decongestant such as xylometazoline; an anti-hypertensive agent such as p-amino clonidine; an anti-glaucoma agent such as a prostaglandin analogue (e.g. latanoprost, bimatoprost or travoprost), a beta blocker (e.g. timolol or betaxolol), an alpha agonist (e.g. brimonidine or apraclonidine) or a carbonic anhydrase inhibitor (e.g. dorzolamide or brinzolamide); a neuro-protective agent; an anaesthetic agent such as benzocaine or pramocaine; a muco-secretagogue agent; an angiostatic agent; pilocarpine; a prostaglandin; a dopaminergic antagonist; a protein; a growth factor; a hyaluronate; hyaluronic acid; ipratropium; or albuterol.

In some embodiments, the composition does not contain added methylglyoxal.

While non-pharmaceutical uses are encompassed by the present invention, in particular embodiments, the composition is a pharmaceutical composition.

The composition of the invention may be formulated in any form suitable for the intended use. For example, the composition of the invention may be formulated as an emulsion, cream, lotion, gel, jelly, paste, ointment, solution, salve or suspension; especially as a emulsion, cream, lotion, gel, paste, ointment, solution or suspension as described in, for example, the United States Food and Drug Administration Monograph No. C-DRG-00201 entitled *CDER Data Standards Manual Definitions for Topical Dosage Forms.* In some embodiments, the composition of the invention is a solution.

In exemplary embodiments, the composition is in the form of a nasal spray, nasal lavage, eye drop, eye spray, eye wash, eye ointment, ear wash, ear drop, throat spray, douche, wound dressing, wound dressing hydrator, wound cleansing solution, lung aspirant, lotion or cream. In particular embodiments, the composition is in the form of a nasal spray, nasal lavage, eye drop, eye spray, eye wash, eye ointment, ear wash, ear drop, throat spray, douche, wound dressing, wound dressing hydrator, wound cleansing solution or lung aspirant; especially a nasal spray or an eye drop.

While the composition may be in a single dose unit form, in some embodiments, the composition is in a multiple dose unit form.

In exemplary embodiments, the composition has a shelf-life of at least one month, two months, three months, four months, five months, six months, one year, two years or three years, especially at least six months, one year, two years or three years. That is, in exemplary embodiments, the composition maintains a pH within a desired range and/or resists microbial contamination for a period of at least one month, two months, three months, four months, five months, six months, one year, two years or three years, for example, when stored at room temperature.

The compositions of the invention may be readily prepared by mixing the components. In some cases, the gluconic acid or a pharmaceutically acceptable salt or derivative thereof, excipients and other additives, such as a preservative, is dissolved in the carrier, especially an aqueous carrier, and warmed to about 40°C to about 55°C. The honey or honey analogue is then mixed in and the temperature maintained between about 40°C and about 55°C while mixing for a suitable time period, such as about 20 minutes to one hour. The mixture is allowed to cool and the pH adjusted to 3.9 to 4.1 if necessary.

The composition may then be sterilised by filtration and/or gamma irradiation to remove pollen and remove or kill clostridial spores or other contaminants that may be naturally present in the honey. In some embodiments, the filter may remove particles greater than about 25 µm, particles greater than about 10 µm and/or particles greater than about 5 µm in size.

### 3. Methods of Stabilising pH

The inventor has found that gluconic acid, or a pharmaceutically acceptable salt or derivative thereof can stabilise the pH of a composition comprising honey or a honey analogue. The minimal change in pH over time is beneficial for the shelf-life of compositions comprising honey or a honey analogue, especially compositions for therapeutic use, such as ocular use, wherein compositions with increased acidity can cause some discomfort upon administration.

Accordingly, in a further aspect, there is provided a method of stabilising the pH of a composition comprising honey or a honey analogue, comprising adding gluconic acid or a pharmaceutically acceptable salt or derivative thereof to the composition.

In particular embodiments, the composition further comprises a carrier, such as a pharmaceutically acceptable carrier. Suitable embodiments of the carrier are discussed in Section 2 *supra.* In particular embodiments, the carrier is an aqueous carrier (e.g. saline or water), glycerol or a combination thereof. The carrier may be added to the composition separately to or simultaneously with the gluconic acid or a pharmaceutically acceptable salt or derivative thereof. For example, the method may further comprise adding a carrier to the composition before or after adding the gluconic acid or a pharmaceutically acceptable salt or derivative thereof. Alternatively, the method may further comprise combining the gluconic acid or a pharmaceutically acceptable salt or derivative thereof with a carrier before the gluconic acid or a pharmaceutically acceptable salt or derivative thereof is added to the composition. For example, the gluconic acid or a pharmaceutically acceptable salt or derivative thereof may be dissolved in a carrier prior to addition to the composition, especially when the carrier is an aqueous carrier.

The amount of honey or honey analogue is discussed in Section 2 *supra.* In some embodiments, the honey or honey analogue is in the composition in an amount in the range of from about 2% to about 98% w/w (and all one tenth integer percentages in between), about 2% to about 90% w/w, about 2% to about 80% w/w, about 2% to about 70% w/w, about 2% to about 60% w/w, about 2% to about 50% w/w, about 2% to about 45% w/w, about 2% to about 40% w/w, about 2% to about 35% w/w, about 2% to about 30% w/w, about 2% to about 25% w/w, about 2% to less than 25% w/w, about 2% to about 20% w/w, about 5% to about 19% w/w, about 7% to about 18% w/w, about 10% to about 18% w/w, about 14 to about 17% w/w or about 15% to about 17% w/w. In particular embodiments, the honey or honey analogue is in an amount of about 16.5% w/w.

Suitable embodiments of the honey or honey analogue are discussed in Section 2 *supra.* In particular embodiments, the composition comprises honey.

Suitable embodiments of the gluconic acid or pharmaceutically acceptable salt or derivative thereof are discussed herein in Section 2. In particular embodiments, the gluconic acid or a pharmaceutically acceptable salt or derivative thereof is added to the composition in an amount in the range of from about 0.05% to about 10% w/w (and all one tenth integer percentages in between), about 0.05% to about 8%, about 0.1% to about 5% w/w, about 0.1% to about 3% w/w, about 0.1% to about 2% w/w, about 0.1% to about 1% w/w, about 0.1% to about 0.7% w/w or about 0.1% to about 0.5% w/w. In particular embodiments, the gluconic acid or a pharmaceutically acceptable salt or derivative thereof is added in an amount of about 0.1%, 0.2%, 0.3% 0.4%, 0.5%, 0.6%, 0.7%, 0.8%, 0.9% or 1% w/w; especially about 0.3% w/w.

In specific embodiments, gluconic acid is added to the composition. In alternative embodiments, a pharmaceutically acceptable salt of gluconic acid is added to the composition, such as sodium gluconate, calcium gluconate, potassium gluconate, magnesium gluconate, zinc gluconate, copper gluconate and ferrous gluconate. In specific embodiments, the pharmaceutically acceptable salt of gluconic acid is sodium gluconate. A derivative of gluconic acid may alternatively be added to the composition, suitable examples of which are discussed above.

In particular embodiments, the pH of the composition is maintained in the range of from about 3 to about 5 (and all one tenth integers in between), about 3.5 to about 4.5, about 3.8 to about 4.2, about 3.9 to about 4.3, or about 3.9 to about 4.1. In particular embodiments, the pH of the composition is maintained at about 3.5, 3.6, 3.7, 3.8, 3.9, 4, 4.1, 4.2, 4.3, 4.4 or 4.5. In some embodiments, the pH of the composition is maintained in any one of the ranges disclosed above for at least one month, two months, three months, four months, five months, six months, one year, two years or three years, especially at least six months, one year, two years or three years, for example, at room temperature.

The method may further comprise adding a further component to the composition. For example, in some embodiments, the method further comprises adding a preservative to the composition. The preservative may be added to the composition before, after or simultaneously with the gluconic acid or a pharmaceutically acceptable salt or derivative thereof and other components of the composition. Suitable embodiments of the preservative are as discussed herein in Section 2.

Other components which may be added to the composition include a tonicity agent, a rheology modifier, a polysaccharide, a surfactant or wetting agent, an oil, a chelating agent, an alcohol, a lubricant (e.g. an ocular lubricant), a cyclodextrin, other natural remedies or plant extracts, an antioxidant and/or a further active agent (e.g. an anti-inflammatory agent, immunosuppressant, antibiotic, anti-viral, anti-protozoal, anti-fungal or anthelmintic). Suitable embodiments of each of these components are discussed in Section 2 *supra.* Each of the components may be added to the composition before, after or simultaneously with the gluconic acid or a pharmaceutically acceptable salt or derivative thereof and other components of the composition. For example, the other component(s) and the gluconic acid or a pharmaceutically acceptable salt or derivative thereof may be combined with a carrier, such as an aqueous carrier, prior to being added to the composition.

In particular embodiments, the composition is a pharmaceutical composition.

The composition may be formulated as an emulsion, cream, lotion, gel, jelly, paste, ointment, solution, salve or suspension as described in Section 2 *supra.* In some embodiments, the composition is a solution.

In exemplary embodiments, the composition is in the form of a nasal spray, nasal lavage, eye drop, eye spray, eye wash, eye ointment, ear wash, ear drop, throat spray, douche, wound dressing, wound dressing hydrator, wound cleansing solution, lung aspirant, lotion or cream. In particular embodiments, the composition is in the form of a nasal spray, nasal lavage, eye drop, eye spray, eye wash, eye ointment, ear wash, ear drop, throat spray, douche, wound dressing, wound dressing hydrator, wound cleansing solution or lung aspirant; especially a nasal spray or an eye drop.

Also provided herein, in another aspect, is a method for maintaining the pH of a composition comprising honey or a honey analogue in a range of about 3.5 to about 4.5, comprising adding gluconic acid or a pharmaceutically acceptable salt or derivative thereof to the composition.

Suitable embodiments of the composition and components thereof are as discussed *supra.* In particular embodiments, the composition comprises honey.

In some embodiments, the pH is maintained in a range of about 3.9 to about 4.1.

In particular embodiments, the pH of the composition is maintained in the specified range for at least one month, two months, three months, four months, five months, six months, one year, two years or three years, especially at least six months, one year, two years or three years.

### 4. Methods of Use

Honey is known to be useful for a range of purposes due to, for example, antimicrobial activity. As such, the inventor has conceived that the compositions of the invention may be used for treating or inhibiting the development of an infection or an inflammatory condition, or for treating a wound.

In a further aspect of the invention, there is provided a method of treating or inhibiting the development of an infection in a subject, comprising administering a composition of the invention to the subject. Also provided is a composition of the invention for use in treating or inhibiting the development of an infection in a subject, a use of a composition of the invention for treating or inhibiting the development of an infection in a subject, and a use of a composition of the invention in the manufacture of a medicament for treating or inhibiting the development of an infection in a subject.

Suitable infections include, but are not limited to, a bacterial, viral, protozoan, fungal or helminth infection; especially a bacterial or a viral infection; most especially a viral infection.

Exemplary bacterial infections include those caused by *Neisseria* species (e.g. *N. meningitidis* and *N. gonorrhoeae*)*, Salmonella* species (e.g. *S*. *enterica* and *S*. *bongori*)*, Streptococcus* species (e.g. *S. pyogenes, S. pneumoniae, S. mitis, S. agalactiae, S. dysgalactiae, S. gallolyticus, S. anginosus, S. sanguinis and S. mutans), Legionella* species (e.g. *L. pneumophila*)*, Mycoplasma* species (e.g. *M. pneumoniae, M. hominis* and *M. genitalum*)*, Bacillus* species (e.g. *B. anthracis* and *B. cereus*)*, Staphylococcus* species (e.g. *S. aureus* and *S*. *epidermis*)*, Chlamydia* species (e.g. *C*. *trachomatis, C. pneumoniae* and *C*. *psittacci*)*, Acinetobacter* species (e.g. *A. baumannii*)*, Actinomadura* species, *Actinomyces* species (e.g. *A. israelii*)*, Anabaena* species, *Anaplasma* species, *Arcanobacterium* species (e.g. *A. haemolyticum*)*, Bacteroides* species (e.g. *B. fragilis*)*, Bdellovibrio* species (e.g. *B. bacteriovorus*)*, Bordetella* species (e.g. *B. pertussis*)*, Borrelia* species (e.g. *B. burgdorferi*)*, Brucella* species (e.g. *B. melitensis*)*, Burkholderia* species (e.g. *B. cepacia, B. pseudomallei, B. xenovorans* and *B. mallei*)*, Campylobacter* species (e.g. *C. jejuni* and *C*. *coli*)*, Caulobacter* species (e.g. *C*. *crescentus*)*, Chlrorbium* species, *Chromatium* species, *Clostridium* species (e.g. *C*. *difficile, C. botulinum, C. perfringens, C. tetani* and *C*. *sordellii*)*, Corynebacterium* species (e.g. *C*. *diptheriae* and *C*. *pseudotuberculosis*)*, Coxiella* species (e.g. C. *burnetti*)*, Cytophaga* species, *Deinococcus* species, *Ehrlichia* species (e.g. *E. chaffeensis* and *E. ewingii*)*, Enterococcus* species (e.g. *E. faecalis* and *E. faecium*)*, Escherichia* species (e.g. *E. coli*)*, Francisella* species (e.g. *F*. *tularensis, F. novicida* and *F. philomiragia*)*, Fusobacterium* species, *Helicobacter* species (e.g. *H. pylori*)*, Haemophilus* species (e.g. *H. influenzae* and *H. ducreyi*)*, Hyphomicrobium* species, *Kingella* species (e.g. *K. kingae*)*, Klebsiella* species (e.g. *K. pneumoniae and K. ozaenae*)*, Leptospira* species (e.g. *L. interrogans*)*, Listeria* species (e.g. *L*. *monocytogenes*)*, Micrococcus* species (e.g. *M. luteus*)*, Myxococcus* species, *Nitrobacter* species, *Nocardia* species (e.g. *N. asteroids, N. brasiliensis* and *N. caviae*)*, Oscillatoria* species, *Pasteurella* species (e.g. *P. multocida*)*, Prochloron* species, *Prevotella* species (e.g. *P. intermedia, P. nigrescens* and *P. copri*)*, Proteus* species (e.g. *P. mirabilis*)*, Pseudomonas* species (e.g. *P. aeruginosa, P. oryzihabitans* and *P. plecoglossicida*)*, Rhodospirillum* species, *Rickettsia* species (e.g. *R. typhi, R. rickettsia, R. akari, R. conorii, R. sibirica, R. australis, R. felis, R. japonica, R. africae* and *R. prowazekii*)*, Shigella* species (e.g. *S*. *dysenteriaw, S. flexneri, S. boydii* and *S*. *sonnei*)*, Spirillum* species, *Spirochaeta* species, *Streptobacillus* species (e.g. *S. moniliformis* and *S*. *minus*)*, Streptomyces* species, *Thiobacillus* species, *Treponema* species (e.g. *T. pallidum*)*, Ureaplasma* species (e.g. *U. urealyticum*)*, Vibrio* species (e.g. *V. cholera, V. parahaemolyticus* and *V. vulnificus*), *Yersinia* species (e.g. *Y. pestis* and *Y. enterocolitica*)*,* and *Mycobacterium* species (e.g. *M*. *tuberculosis, M. africanum, M. canetti, M. microti* and *M. leprae*)*.*

Suitable viral infections include, but are not limited to, infections caused by Picornaviruses (e.g. hepatitis A virus, enteroviruses such as poliovirus, enterovirus 71, 70, 69, and 68, Coxsackieviruses, echoviruses, foot and mouth disease virus, and rhinoviruses), Caliciviruses (e.g. hepatitis E virus, noroviruses such as Norwalk virus, feline calicivirus), Arteriviruses (e.g. equine arteritis virus), Togaviruses (e.g. sindbis virus, the equine encephalitis viruses, chikungunya virus, rubella virus, Ross River virus, bovine diarrhea virus, hog cholera virus, Semliki forest virus), Flaviviruses (e.g. dengue virus, West Nile virus, yellow fever virus, Japanese encephalitis virus, St. Louis encephalitis virus, tick-borne encephalitis virus, bovine viral diarrhea virus, classical swine fever virus), Coronaviruses (e.g. human coronaviruses, including betacoronaviruses such OC43 and HKU1 of the A lineage, SARS-CoV and SARS-CoV-2 of the B lineage and MERS-CoV of the C lineage, swine gastroenteritis virus), Rhabdoviruses (e.g. rabies virus, Australian bat lyssavirus, vesicular stomatitis viruses), Filoviruses (e.g. Marburg virus, Ebola virus), Paramyxoviruses (e.g. measles virus, canine distemper virus, mumps virus, parainfluenza viruses, respiratory syncytial virus, Newcastle disease virus, rinderpest virus, Nipah virus, Hendra virus), Orthomyxoviruses (e.g. human influenza viruses, including human influenza virus types A, B and C, avian influenza viruses, equine influenza viruses), Bunyaviruses (e.g. hantavirus, LaCrosse virus, Rift Valley fever virus), Arenaviruses (e.g. Lassa virus, Machupo virus), Reoviruses (e.g. human and animal reoviruses, such as rotaviruses, bluetongue virus), Birnaviruses (e.g. infectious bursal virus, fish pancreatic necrosis virus), Retroviruses (e.g. HIV 1, HIV 2, HTLV-1, HTLV-2, bovine leukemia virus, feline immunodeficiency virus, feline sarcoma virus, mouse mammary tumor virus), Hepadnaviruses (e.g. hepatitis B virus), Parvoviruses (e.g. B19 virus, canine parvovirus, feline panleukopenia virus), Papovaviruses (e.g. human papillomaviruses, SV40, bovine papillomaviruses), Adenoviruses (e.g. human, canine, bovine, and porcine adenoviruses), Herpesviruses (e.g. herpes simplex viruses, varicella-zoster virus, infectious bovine rhinotracheitis virus, cytomegalovirus, human herpesvirus 6, human herpesvirus 7, human herpesvirus 8, Epstein-Barr virus), Poxviruses (e.g. vaccinia, fowlpoxviruses, raccoon poxvirus, skunkpox virus, monkeypoxvirus, cowpox virus, buffalopox virus, musculum contagiosum virus), human T-cell lymphotropic virus, small pox virus, polyoma virus, junin virus, an astrovirus, BK virus, machupo virus, sabia virus, sapovirus, a coltivirus, bocavirus, human metapneumovirus, lymphocytic choriomeningitis mammarenavirus, guanarito mammarenavirus, molluscum contagiosum virus and JC virus; especially infections caused by a coronavirus, such as SARS-CoV-2.

Suitable helminth infections include, but are not limited to, infections caused by *Ascaris* species, *Baylisascaris* species, *Clonorchis* species, *Taenia* species, *Diphyllobothrium* species, *Dracunculus* species, *Echinococcus* species, *Enterobius* species, *Fasciolopsiasis* species, *Fasciola* species, *Wuchereria* species, *Brugia* species, *Gnathostoma* species, *Ancylostoma* species, *Hymenolepis* species, *Metagonimiasis* species, *Onchocerciasis* species, *Paragonimus* species, *Schistosoma* species, *Strongyloides* species, *Toxocara* apecies, *Trichinella* species and *Trichuris* species.

The protozoan infection may include, but is not limited to, malaria (e.g. an infection caused by *Plasmodium falciparum, Plasmodium malariae, Plasmodium ovale, Plasmodium vivax* or *Plasmodium knowlesi*)*,* or an infection caused by *Entamoeba histolytica, Balantidium coli, Blastocystis* species, *Cyclospora cayetanensis, Acanthamoeba* species, *Balamuthia* species, *Naegleria* species, *Leishmania* species, *Sappinia* species, *Giardia* species, *Isospora* species, *Rhinosporidium* species, *Toxoplasma* species, *Trichomonas* species, *Trypanosoma* species (e.g. *T. brucei* or *T. cruzi*)*, Babesia* species, *Cryptosporidium* species or *Theileria* species.

Exemplary fungal infections include, but are not limited to, infections caused by *Candida* species (e.g. *C*. *parapsilosis, C. famata, C. krusei, C. albicans, C. glabrata* and *C*. *tropicalis*)*, Aspergillus* species, *Blastomyces* species, *Coccidioides* species, *Cryptococcus* species, *Trichophyton* species, *Microsporum* species, *Geotrichum* species, *Histoplasma* species, *Madurella* species, *Paracoccidioides* species, *Pneumocystis* species (e.g. *P. carinii*)*, Sporothrix* species, *Nannizzia* species, *Rhizopus* species, *Mucor* species, *Rhizomucor* species and *Syncephalastrum* species.

In particular embodiments, the infection is a pathogenic infection, especially a viral infection.

The infection may be an acute or chronic infection.

In particular embodiments, the infection is an ophthalmic infection, vaginal infection, nasal infection, ear infection, respiratory tract infection including lung infection, sinus infection, throat infection, skin infection or wound infection; especially an ophthalmic infection or a nasal infection.

The infection may, in some embodiments, be associated with cystic fibrosis, tuberculosis or AIDS.

Further provided is a method of killing or inhibiting the growth of an infectious agent, comprising contacting the infectious agent with a composition of the invention; a composition of the invention for use in killing or inhibiting the growth of an infectious agent, wherein the infectious agent is contacted with the composition of the invention; a use of a composition of the invention for killing or inhibiting the growth of an infectious agent, wherein the infectious agent is contacted with the composition of the invention; and a use of a composition of the invention in the manufacture of a medicament for killing or inhibiting the growth of an infectious agent, wherein the infectious agent is contacted with the composition of the invention.

In particular embodiments, the infectious agent has infected a subject, such as a human subject.

Suitable infectious agents include a bacteria, virus, protozoa, fungus or helminth. Representative bacteria, viruses, protozoans, fungi and helminths include those discussed above.

Also contemplated by the present invention is a method of treating or inhibiting the development of a condition associated with an infection in a subject, comprising administering a composition of the invention to a subject. Further provided is a use of the composition of the invention for treating or inhibiting the development of a condition associated with an infection in a subject, a composition of the invention for use in treating or inhibiting the development of a condition associated with an infection in a subject, and a use of a composition of the invention in the manufacture of a medicament for treating or inhibiting the development of a condition associated with an infection in a subject.

In specific embodiments, the condition is an ophthalmic, respiratory, nasal, ear, skin or vaginal condition, or is an allergic reaction.

Exemplary conditions include sinusitis or rhinosinusitis (including acute rhinosinusitis, recurrent acute rhinosinusitis, subacute rhinosinusitis, chronic rhinosinusitis and acute exacerbation of chronic rhinosinusitis), rhinitis, pharyngitis, tonsillitis, laryngitis, blepharitis, stye, conjunctivitis (including trachoma), keratitis, kerato-conjunctivitis, rhinoconjunctivitis, acanthamoeba keratitis, otitis (e.g. otitis externa, otitis media or otitis interna), vaginitis (e.g. bacterial vaginosis), vaginal candidiasis, herpes simplex virus infections or sexually transmitted infections or diseases (e.g. chlamydia and gonorrhoea).

In some embodiments, the condition is an inflammatory condition.

In another aspect, there is provided a method of treating or inhibiting the development of an inflammatory condition in a subject, comprising administering a composition of the invention to the subject. Also provided is a use of a composition of the invention for treating or inhibiting the development of an inflammatory condition in a subject, a composition of the invention for use in treating or inhibiting the development of an inflammatory condition in a subject, and a use of a composition of the invention in the manufacture of a medicament for treating or inhibiting the development of an inflammatory condition in a subject.

The inflammatory condition may be any condition associated with or involving inflammation. In particular embodiments, the condition is associated with and/or involves inflammation of an epithelial lined surface, especially a mucosal epithelial lined surface.

In particular embodiments, the inflammatory condition is an ocular condition. Exemplary conditions include, but are not limited to, blepharitis, dry eye syndrome, stye, conjunctivitis (including trachoma), keratitis, kerato-conjunctivitis, ocular herpes simplex virus infection, Fuch's dystrophy, acanthamoeba keratitis, corneal oedema, allergic conjunctivitis and rhinoconjunctivitis; especially blepharitis, dry eye syndrome or corneal oedema.

In alternative embodiments, the inflammatory condition is a nasal condition. Suitable nasal conditions include, but are not limited to, rhinitis, including infective, allergic and nonallergic (vasomotor) rhinitis. The inflammatory condition may also be a sinus condition, such as sinusitis or rhinosinusitis (including acute rhinosinusitis, recurrent acute rhinosinusitis, subacute rhinosinusitis, chronic rhinosinusitis and acute exacerbation of chronic rhinosinusitis).

The inflammatory condition may also be a condition of the respiratory tract, such as pharyngitis, tonsillitis or laryngitis.

In some embodiments, the inflammatory condition is an ear condition, such as otitis (e.g. otitis externa, otitis media or otitis interna).

The inflammatory condition may also be a vaginal condition, such as vaginitis (e.g. bacterial vaginosis, vaginal candidiasis or trichomoniasis).

In particular embodiments, the inflammatory condition is a skin condition. For example, suitable conditions include, but are not limited to, eczema, contact dermatitis or urticaria, especially eczema.

The inflammatory condition may also be inflammation associated with an infection, suitable examples of which are described elsewhere herein. Alternatively, the inflammatory condition may be inflammation associated with a trauma, such as surgery or another physical trauma.

In some embodiments, the inflammatory condition is an allergic reaction. The allergic reaction may be any reaction in response to a foreign substance or event, such as an infectious agent or component thereof (e.g. virus, bacteria, protozoa, fungi or helminth), an airborne allergen (e.g. pollen, animal dander, dust mite and mould spores), an insect bite (e.g. ant, mosquito and the like), an insect sting (e.g. bee and wasp), an arachnid bite (e.g. spider, scorpion, tick, mite, such as a Demodex mite, and the like), a leech bite, a medication or vaccine, a substance which has come into contact with the skin (e.g. a plant or plant substance, an insect or insect substance including a caterpillar, moth or butterfly and substances thereof, latex and the like) or a food allergen (e.g. peanut, egg, milk and the like). In particular embodiments, the allergic reaction is associated with an insect bite or sting, an arachnid bite or an infection; especially an insect bite or an infection. Suitable infectious agents and infections are described above.

In particular embodiments, the inflammatory condition involves inflammation of an epithelial lined surface, especially a mucosal epithelial lined surface. The epithelial lined surface may include, but is not limited to, the outside or inside cavities or lumen of bodies such as the skin, tissue lining the mouth, nose, sinus, oesophagus or lungs; tissue lining the rectum or anus; tissue lining the vagina or urethra; tissue lining the outer surface of the cornea; or tissue lining the ears. A mucosal epithelial lined surface includes, but is not limited to, the surface of the respiratory tract, including the sinus, mouth, nose and oesophagus; or the surface of the ears, eyes, genitals or anus.

The compositions of the invention are also proposed to be useful for modulating an inflammatory response.

The inflammatory response may be triggered or initiated by a number of stimuli, including, but not limited to, an infection or infectious agent (examples of which are described above), an allergen (e.g. an allergen from an infectious agent, pollen, animal dander, a dust mite, a Demodex mite, mould spores, an insect bite or sting, an arachnid bite or sting, a plant, a medication or vaccine, a food allergen and the like), an insect or arachnid bite or sting or a self-antigen.

While any subject is contemplated, in some embodiments, the subject is suffering from an infection, suitable examples of which are discussed *supra.*

Also provided is a method of modulating the development of inflammation in a subject, comprising administering a composition of the invention to the subject. The inflammation may be, for example, associated with an infection, an allergic reaction, a trauma (such as surgery) or is an ophthalmic, respiratory, nasal, ear, skin or vaginal condition. Suitable examples of infections, allergic reactions and ophthalmic, respiratory, nasal, ear, skin and vaginal conditions are described above.

In another aspect, there is provided a method of treating or inhibiting the development of an ophthalmic condition in a subject, comprising administering a composition of the invention to the subject. Also provided is a use of a composition of the invention for treating or inhibiting the development of an ophthalmic condition in a subject, a composition of the invention for use in treating or inhibiting the development of an ophthalmic condition in a subject, and a use of a composition of the invention in the manufacture of a medicament for treating or inhibiting the development of an ophthalmic condition in a subject.

Suitable ophthalmic conditions include, but are not limited to, blepharitis, dry eye syndrome, stye, conjunctivitis (including trachoma, allergic conjunctivitis or rhinoconjunctivitis), keratitis, kerato-conjunctivitis, herpes simplex virus infection, Fuch's dystrophy, Sjögren's syndrome, non-Sjögren's syndrome, acanthamoeba keratitis, corneal oedema, especially blepharitis or dry eye syndrome.

In another aspect, there is provided a method of treating or inhibiting the development of a nasal or sinus condition in a subject, comprising administering a composition of the invention to the subject. Also provided is a use of a composition of the invention for this purpose, a composition of the invention for use in this purpose, and a use of a composition of the invention in the manufacture of a medicament for this purpose.

In particular embodiments, the nasal or sinus condition is sinusitis or rhinosinusitis (including acute rhinosinusitis, recurrent acute rhinosinusitis, subacute rhinosinusitis, chronic rhinosinusitis and acute exacerbation of chronic rhinosinusitis), or rhinitis (including infective, allergic and nonallergic (vasomotor) rhinitis).

The composition of the invention may be used for treating or inhibiting the development of blepharitis, dry eye syndrome, corneal oedema, rhinitis or sinusitis; especially blepharitis, dry eye syndrome, rhinitis or sinusitis
In any one of the above aspects, the blepharitis may have developed as a result of, for example, any one or more of a bacterial infection of the eyelids, typically with a *Staphylococcus* species; mite infestation (e.g. a Demodex mite); a skin condition, such as seborrhoeic dermatitis or skin rosacea; meibomian gland dysfunction and any combination thereof. The dry eye syndrome may have occurred as a result of, for example, any one or more of age; hormonal changes; autoimmune diseases, such as primary Sjögren's syndrome, rheumatoid arthritis, Stevens-Johnson syndrome, cicatricial pemphigoid or lupus erythematosus; medication use, such as use of antihistamines, antidepressants, beta-blockers, oral isotretinoin and oral contraceptives; lifestyle factors resulting in decreased blinking, such as tasks which require close visual attention; certain conditions resulting in an impaired ability to blink such as stroke or Bell's palsy; chemical burns to the eye; meibomian gland dysfunction; meibomian gland disease; rosacea; an infection; blepharitis; or trauma, such as surgery. In some embodiments, the corneal oedema has developed as a result of eye surgery such as cataract surgery or a corneal transplant, infection such as herpes simplex virus infection, glaucoma or Fuch's dystrophy. In certain embodiments, rhinitis includes, but is not limited to, infective, allergic and nonallergic (vasomotor) rhinitis. In particular embodiments, sinusitis or rhinosinusitis includes, but is not limited to, acute rhinosinusitis, recurrent acute rhinosinusitis, subacute rhinosinusitis, chronic rhinosinusitis or acute exacerbation of chronic rhinosinusitis.

In a further aspect, there is provided a method of treating or inhibiting the development of a condition associated with inflammation or infection, especially inflammation or infection of an epithelial lined surface, especially a mucosal epithelial lined surface. The epithelial lined surface may include, but is not limited to, the outside or inside cavities or lumen of bodies such as the skin, tissue lining the mouth, nose, sinus, oesophagus or lungs; tissue lining the rectum or anus; tissue lining the vagina or urethra; tissue lining the outer surface of the cornea; or tissue lining the ears. A mucosal epithelial lined surface includes, but is not limited to, the surface of the respiratory tract, including the sinus, mouth, nose and oesophagus; or the surface of the ears, eyes, genitals or anus. Suitable conditions are discussed *supra.*

Also contemplated is the use of the composition of the invention for lubricating an eye of a subject. This may be useful, for example, for treating or inhibiting the development of an ophthalmic condition, such as, but not limited to, dry eye syndrome, Sjögren's syndrome, non-Sjögren's syndrome or corneal oedema. In such uses, the composition may be administered via topical ocular administration.

In particular embodiments, the composition may be used to provide an artificial tear composition or may supplement an artificial tear composition.

The composition of the invention may also be used for treating a wound and/or inhibiting the development of an infection or inflammation resulting from a wound. Accordingly, in another aspect of the invention, there is provided a method of hydrating a wound or a wound dressing, comprising contacting the dressing with a composition of the invention. Also provided is a method of hydrating a dressing, comprising contacting the dressing with a composition of the invention.

Examples of dressings include wound dressings, sanitary dressings and breast pads. In some embodiments sanitary dressings include absorbent sanitary pads, napkins and tampons. Suitable sanitary dressings may include, but are not limited to, dressings comprising cotton, rayon, bleached wood pulp, sphagnum and polyacrylate gels. Suitable breast pads may include, but are not limited to, pads comprising bamboo, cotton and rayon.

In some embodiments, the wound dressing is an absorbent dressing. Suitable wound dressings include, but are not limited to, gauze including acetate gauze or Telfa dressings, sponge, pads, packing strips, tulle and foam dressings, gels or hydrocolloid sheets. The wound dressing may be a primary or secondary dressing. In particular embodiments, the wound dressing is a hydrocolloid dressing.

In another aspect, there is provided a method of hydrating or cleaning a wound, comprising contacting the wound with a composition of the invention. In particular embodiments, the wound may be contacted with a composition of the invention by washing, rinsing, flushing, lavage or irrigation.

The wound may be a superficial, partial or full thickness wound. In exemplary embodiments, the wound is a surgical incision, burn, cut, scrape, abrasion, venous stasis or ulceration (e.g. a pressure ulcer or diabetic skin ulcer). In particular embodiments, the hydrated wound dressing is used to soften necrotic tissue. In further embodiments, the hydrated wound dressing is used to remove necrotic tissue.

In any one of the above aspects, the composition may alleviate the symptoms of a condition, such as an infection, an inflammatory condition or a respiratory, nasal, sinus, ophthalmic, ear, skin or vaginal condition. For example, in some embodiments, the composition of the invention alleviates the symptoms of dry eye syndrome, blepharitis, rhinitis or sinusitis without necessarily curing the condition. In some embodiments, the composition of the invention reduces inflammation that may be present in a subject having an infection, inflammatory condition or a respiratory, nasal, sinus, ophthalmic, ear, skin or vaginal condition.

Any one of the aspects described above may involve administration of an effective amount of the composition of the invention. The composition of the invention may be administered via any suitable route of administration, such as oral, topical, intranasal, intraocular, auricular, transmucosal, intravaginal, inhalational or intraperitoneal administration. In particular embodiments, the composition is administered via topical, intraocular, intranasal or inhalational administration; especially topical (e.g. topical ocular administration) or intranasal administration.

For example, ophthalmic compositions may be applied topically to the eye as a wash, drop, irrigation, flush, rinse, ointment or lavage. The composition may be applied to the ear, for example, as a drop, wash, lavage, irrigation, flush, rinse or may be syringed. The composition of the invention may be applied to the vagina as a douche. The composition may be administered to the respiratory tract by flushing, washing, lavage, irrigation, rinsing, drops, aspirant or spray. Intranasal administration of the composition may be achieved via a spray, drops, lavage, irrigation, rinsing, washing, flushing or aspirant.

In some embodiments, the composition of the invention may be administered by more than one route of administration concurrently, for example, eye drops and nasal spray. For example, in some embodiments, the composition of the invention may be used to treat or prevent more than one condition concurrently. For example, the compositions of the invention may be used to treat or prevent an ophthalmic condition and a respiratory condition concurrently, such as dry eye syndrome and rhinosinusitis or sinusitis. This may be achieved, for example, by administering the composition in the form of eye drops and nasal spray concurrently. In exemplary embodiments, the composition of the invention is used to treat or inhibit the development of rhinoconjunctivitis or allergic conjunctivitis and/or rhinosinusitis.

The dosage and frequency will depend on the subject, the condition, disease or disorder to be treated and the route of administration. A skilled person will readily be able to determine suitable dosages and frequency of such dosages. For example, treatment for ophthalmic conditions may include about 2 to 8 drops per eye, treatment for the respiratory tract or a nasal condition may include about 2 to 4 sprays and treatment for the ear may include about 2 to 20 drops in the affected ear. The composition may be administered at a frequency of, for example, once, twice or three times daily. The treatment may be continued for multiple days, weeks, months or years. In embodiments where the composition comprises one or more additional active agents, the dosages and frequency of administration are determined by reference to the usual dose and manner of administration of the said agents.

Any one of the methods described above may, in some embodiments, involve the administration of one or more further active agents as described in Section 2 *supra,* such as an anti-inflammatory agent, immunosuppressant, antibiotic, anti-viral, anti-protozoal, anti-fungal or anthelmintic.

In order that the invention may be readily understood and put into practical effect, particular preferred embodiments will now be described by way of the following non-limiting examples.

### EXAMPLES

Starting materials, reagents and equipment used are commercially available (e.g. from Sigma-Aldrich, Burlington, Massachusetts, USA and the like) and were obtained from commercial sources unless otherwise indicated.

### EXAMPLE 1 - PREPARATION OF HONEY COMPOSITIONS

Prior to incorporation, raw honey was processed by blending to ensure homogeneity, preferably using a low shear mixer. The honey is then heated to dissolve any crystals that may have formed during storage. Preferably the honey is heated to temperatures of less than 75°C. The honey is filtered to remove undissolved crystals and any extraneous matter. Preferably the honey is filtered through a 5-10 µm filter using pressure filtration. Prior to filtration, the honey may be centrifuged to aid the removal of wax.

A composition comprising 16.5% w/w honey was prepared (Honey Composition A). The components of the composition are provided in Table 1. The water was heated to between 45 and 55°C, and sodium benzoate was dissolved in the heated water. When the sodium benzoate was dissolved, honey, sodium chloride and glycerine was added to the solution and the solution was stirred for 45 mins at a temperature of between 45 and 55°C. The solution was then cooled to between 40 and 45°C and filtered through a 5 µm filter, before being subjected to gamma radiation.

**TABLE 1**

| **HONEY COMPOSITION A** | | |
|---|---|---|
| **Ingredient** | **Quantity (mg/g)** | **Quantity (% w/w)** |
| Honey | 165 | 16.5% |
| Purified water | 822.8 | 82.28% |
| Sodium benzoate (benzoic acid in finished product of 0.1% w/w) | 1.2 | 0.12% |
| Sodium chloride | 9 | 0.9% |
| Glycerine | 2 | 0.2% |

A composition comprising 98% w/w honey was prepared (Honey Composition B). The components of the composition are provided in Table 2. Honey and glycerine were mixed and heated to a temperature of between 55 and 65°C. The solution was then cooled to between 40 and 45°C and filtered through a 5 µm filter, before being subjected to gamma radiation.

**TABLE 2**

| **HONEY COMPOSITION B** | | |
|---|---|---|
| **Ingredient** | **Quantity (mg/g)** | **Quantity (% w/w)** |
| Honey | 980 | 98% |
| Purified water | 18 | 1.8% |
| Glycerine | 2 | 0.2% |

### EXAMPLE 2 - STABILITY OF PH OF HONEY COMPOSITION IN PRESENCE OF SALTS

The stability of the pH of Honey Composition A over time in the presence of sodium acetate, sodium citrate or sodium gluconate was assessed in comparison to Honey Composition A alone (prepared in accordance with Example 1), and Honey Composition B (prepared in accordance with Example 1). 0.1M sodium acetate, sodium citrate and sodium gluconate buffers (in water) were prepared and the pH of the buffers was measured (refer to Table 3). For buffer addition, Honey Composition A was prepared in accordance with Example 1 but was not subjected to gamma irradiation. The composition was heated to between 40 and 45°C, and the sodium acetate, sodium citrate or sodium gluconate buffer was added to the composition in accordance with Table 4 in an amount resulting in a pH of approximately 4.3. The composition was then subjected to gamma radiation.

**TABLE 3**

| **BUFFER PREPARATION** | | |
|---|---|---|
| **Buffer** | **Mass of Salt Used (g/L water)** | **pH after Preparation** |
| Sodium acetate | 8.2030 | 8.23 |
| Sodium citrate | 29.41 | 8.79 |
| Sodium gluconate | 21.814 | 7.44 |

**TABLE 4**

| **SAMPLE PREPARATION** | | | | | |
|---|---|---|---|---|---|
| **Sample** | **Volume of buffer (mL)** | **Volume of Honey Composition A (mL)** | **pH After Buffer Addition** | **pH 24 h After Buffer Addition** | **pH After Irradiation** |
| Lot 1 (sodium acetate buffer) | 40 | 900 | 4.3 | 4.29 | 4.12 |
| Lot 2 (sodium citrate buffer) | 20 | 900 | 4.3 | 4.31 | 4.12 |
| Lot 3 (sodium gluconate buffer) | 150 | 900 | 4.3 | 4.34 | 4.23 |

Following buffer addition, the compositions were stored at 45°C for up to nine months. The pH was measured each month using a three point calibrated pH meter and the moisture content was measured each month using Karl Fischer titration (AOAC Method 967.19). The 5-hydroxymethylfurfural (HMF) content was measured every three months using spectrophotometry according to the British Pharmacopoeia Honey monograph (Ph. Eur. Monograph 2051). The results are presented in Table 5.

**TABLE 5**

| **PH, MOISTURE CONTENT AND HMF CONTENT IN HONEY COMPOSITION A ALONE OR WITH SODIUM ACETATE, SODIUM CITRATE OR SODIUM GLUCONATE** | | | | | | | |
|---|---|---|---|---|---|---|---|
| **Measurement** | **Sample** | **Time (months)** | | | | | |
| | | **0M** | **1M** | **2M** | **3M** | **4M** | **5M** |
| pH | Lot 1 | 4.04 | 3.83 | 3.76 | 3.72 | 3.65 | 3.67 |
| | Lot 2 | - | 3.79 | 3.76 | 3.73 | 3.66 | 3.68 |
| | Lot 3 | - | 3.97 | 3.96 | 3.94 | 3.87 | 3.91 |
| | Honey A | - | 3.56 | 3.39 | 3.39 | 3.36 | 3.27 |
| | Honey B | 3.73 | 3.67 | 3.49 | 3.48 | 3.43 | 3.28 |
| Moisture (%) | Lot 1 | 89.04 | 88.72 | 84.73 | 85.78 | 85.79 | 85.19 |
| | Lot 2 | - | 88.99 | 84.69 | 86.67 | 87.68 | 85.26 |
| | Lot 3 | - | 90.96 | 86.13 | 87.24 | 86.98 | 88.90 |
| | Honey A | - | 85.47 | 87.26 | 84.72 | 84.77 | 84.21 |
| | Honey B | 17.47 | 19.64 | 27.12 | 19.29 | 21.10 | 19.97 |
| HMF (ppm) | Lot 1 | 171.92 | | | 690.12 | | |
| | Lot 2 | - | | | 649.70 | | |
| | Lot 3 | - | | | 595.81 | | |

| **Measurement** | **Sample** | **Time (months)** | | | | | |
|---|---|---|---|---|---|---|---|
| | | **6M** | **7M** | **8M** | **9M** | | |
| pH | Lot 1 | 3.64 | 3.57 | 3.51 | 3.49 | | |
| | Lot 2 | 3.58 | 3.59 | 3.51 | 3.49 | | |
| | Lot 3 | 3.77 | 3.82 | 3.75 | 3.75 | | |
| | Honey A | - | 3.27 | - | 3.18 | | |
| | Honey B | - | 3.8 | - | 3.18 | | |
| Moisture (%) | Lot 1 | 87.32 | 86.16 | 88.11 | 85.67 | | |
| | Lot 2 | 88.13 | 85.67 | 86.47 | 84.99 | | |
| | Lot 3 | 88.60 | 86.91 | 89.74 | 88.19 | | |
| | Honey A | - | 85.39 | - | 86.62 | | |
| | Honey B | - | 19.67 | - | 21.30 | | |
| HMF (ppm) | Lot 1 | 1694.60 | | | 2146.70 | | |
| | Lot 2 | 1880.23 | | | 2329.33 | | |
| | Lot 3 | 1790.41 | | | 2344.30 | | |

Wherein Honey A is Honey Composition A and Honey B is Honey Composition B.

As is evident from Table 5, the pH of Honey Composition A (16.5% w/w honey) and Honey Composition B (98% w/w honey) became more acidic over time, changing from pH 4.04 and 3.73, respectively, to pH 3.18 over a period of nine months at 45°C. The addition of sodium acetate and sodium citrate (Lots 1 and 2, respectively) to Honey Composition A resulted in a slight reduction in the pH change over time, with the pH changing from pH 4.04 to pH 3.49 over a period of nine months at 45°C. The addition of sodium gluconate (Lot 3), however, resulted in a significant stabilisation of the pH, with the pH changing from pH 4.04 to pH 3.75 after nine months at 45°C. Surprisingly, the pH of this composition changed by only about 0.13 after five months at 45°C.

The preservative efficacy of the composition of Lot 3 was also determined after nine months of storage at 45°C using the method outlined in the Guidelines of British Pharmacopoeia 2021 Appendix XVI C for effective antimicrobial preservation for an eye preparation. Bacteria were cultured in soya agar at a temperature of 30-35 °C for two days prior to addition to the composition of Lot 3. Fungi were cultured in Sabouraud dextrose agar at a temperature of 20-25 °C for three to five days prior to addition to the composition of Lot 3. Samples were challenged with *Pseudomonas aeruginosa, Staphylococcus aureus, Candida albicans* and *Aspergillus brasiliensis* by adding inoculum to the composition of Lot 3 after nine months of storage at 45°C and recovering microorganisms from the inoculated sample. The samples were challenged with a single inoculum of the test microorganism. The amount of microorganisms present in the test honey samples was determined at defined intervals throughout the study period.

The results are presented in Table 6. The composition of Lot 3 passed Criteria A of the British Pharmacopoeia 2021 Appendix XVI C Criteria for effective antimicrobial preservation for an eye preparation after nine months of storage at 45°C.

**TABLE 6**

| **PRESERVATIVE EFFICACY OF THE COMPOSITION OF LOT 3 AFTER NINE MONTHS OF STORAGE AT 45°C** | | | | | | | | | | | | | |
|---|---|---|---|---|---|---|---|---|---|---|---|---|---|
| Test Organism | 1^{st} Inoculum | | Recovery Counts | | | | | | | | | | |
| | CFU per gram | Log CFU | Time zero CFU per gram | Six hours | | 24 hours | | Seven days | | 14 days | | 28 days | |
| | | | | CFU per gram | Log reduction* | CFU per gram | Log reduction* | CFU per gram | Log reduction* | CFU per gram | Log reduction* | CFU per gram | Log reduction* |
| *S. aureus* ATCC 6538 | 5.7 × 10⁵ | 5.76 | >3 × 10⁵ | 2.1 × 10² | 3.44 | <10 | >4.76 | <10 | >4.76 | - | - | <10 | >4.76 |
| *P*. *aeruginosa* ATCC 9027 | 1 × 10⁶ | 6.00 | 7.5 × 10³ | <10 | >5.00 | <10 | >5.00 | <10 | >5.00 | - | - | <10 | >5.00 |
| *C. albicans* ATCC 10231 | 6.3 × 10⁵ | 5.80 | >3 × 10⁵ | - | - | - | - | <10 | >4.80 | <10 | >4.80 | <10 | >4.80 |
| *A. brasiliensis* ATCC 16404 | 4.6 × 10⁵ | 5.66 | >3 × 10⁵ | - | - | - | - | <10 | >4.66 | <10 | >4.66 | <10 | >4.66 |

| | | | | | | | | | | | | | |
|---|---|---|---|---|---|---|---|---|---|---|---|---|---|
| wherein * = Log reduction from initial count; and CFU = colony forming units | | | | | | | | | | | | | |

The disclosure of every patent, patent application, and publication cited herein is hereby incorporated herein by reference in its entirety.

The citation of any reference herein should not be construed as an admission that such reference is available as "Prior Art" to the instant application.

Throughout the specification the aim has been to describe the preferred embodiments of the invention without limiting the invention to any one embodiment or specific collection of features. Those of skill in the art will therefore appreciate that, in light of the instant disclosure, various modifications and changes can be made in the particular embodiments exemplified without departing from the scope of the present invention. All such modifications and changes are intended to be included within the scope of the appended claims.

### EMBODIMENTS

Exemplary embodiments include, but are not limited to:
1. A composition comprising honey or a honey analogue, gluconic acid or a pharmaceutically acceptable salt or derivative thereof and a pharmaceutically acceptable carrier.
2. The composition according to embodiment 1, wherein the honey or honey analogue is in an amount in the range of from about 2% to about 98% w/w (and all one tenth integer percentages in between).
3. The composition according to embodiment 2, wherein the honey or honey analogue is in an amount in the range of from about 2% to about 50% w/w (and all one tenth integer percentages in between).
4. The composition according to embodiment 2, wherein the honey or honey analogue is in an amount in the range of from about 2% to about 45% w/w (and all one tenth integer percentages in between).
5. The composition according to embodiment 2, wherein the honey or honey analogue is in an amount in the range of from about 7% to about 18% w/w (and all one tenth integer percentages in between).
6. The composition according to embodiment 2, wherein the honey or honey analogue is in an amount of about 16.5% w/w.
7. The composition according to any one of embodiments 1-6, wherein the gluconic acid or a pharmaceutically acceptable salt or derivative thereof is in an amount in the range of from about 0.05% to about 10% w/w (and all one tenth integer percentages in between).
8. The composition according to embodiment 7, wherein the gluconic acid or a pharmaceutically acceptable salt or derivative thereof is in an amount in the range of from about 0.1% to about 5% w/w (and all one tenth integer percentages in between).
9. The composition according to embodiment 7, wherein the gluconic acid or a pharmaceutically acceptable salt or derivative thereof is in an amount in the range of from about 0.1% to about 3% w/w (and all one tenth integer percentages in between).
10. The composition according to embodiment 7, wherein the gluconic acid or a pharmaceutically acceptable salt or derivative thereof is in an amount in the range of from about 0.1% to about 1% w/w (and all one tenth integer percentages in between).
11. The composition according to embodiment 7, wherein the gluconic acid or a pharmaceutically acceptable salt or derivative thereof is in an amount in the range of from about 0.1% to about 0.5% w/w (and all one tenth integer percentages in between).
12. The composition according to embodiment 7, wherein the gluconic acid or a pharmaceutically acceptable salt or derivative thereof is in an amount of about 0.3% w/w.
13. The composition according to any one of embodiments 1-12, wherein the gluconic acid is in the form of a pharmaceutically acceptable salt.
14. The composition according to embodiment 13, wherein the pharmaceutically acceptable salt is selected from the group consisting of sodium gluconate, calcium gluconate, potassium gluconate, magnesium gluconate, zinc gluconate, copper gluconate and ferrous gluconate.
15. The composition according to embodiment 13, wherein the pharmaceutically acceptable salt is sodium gluconate.
16. The composition according to any one of embodiments 1-15, wherein the composition further comprises a preservative.
17. The composition according to embodiment 16, wherein the preservative is selected from the group consisting of benzoic acid, sorbic acid, a flavonoid, a phenolic acid, abscisic acid, ascorbic acid and pharmaceutically acceptable salts and combinations thereof.
18. The composition according to embodiment 16, wherein the preservative is benzoic acid or a pharmaceutically acceptable salt thereof.
19. The composition according to embodiment 16, wherein the preservative is sodium benzoate.
20. The composition according to any one of embodiments 1-19, wherein the pH of the composition is in the range of from about 3.5 to about 4.5 (and all one tenth integers in between).
21. The composition according to embodiment 20, wherein the pH of the composition is in the range of from about 3.9 to about 4.1 (and all one tenth integers in between).
22. The composition according to any one of embodiments 1-21, wherein the composition is a pharmaceutical composition.
23. The composition according to any one of embodiments 1-22, wherein the composition is in the form of a nasal spray, nasal lavage, eye drop, eye spray, eye wash, eye ointment, ear wash, ear drop, throat spray, douche, wound dressing, wound dressing hydrator, wound cleansing solution or lung aspirant.
24. The composition according to embodiment 23, wherein the composition is in the form of a nasal spray or an eye drop.
25. The composition according to any one of embodiments 1-24, wherein the carrier is an aqueous carrier, glycerol or a combination thereof.
26. The composition according to embodiment 25, wherein the aqueous carrier is saline or water.
27. The composition according to any one of embodiments 1-26, wherein the composition comprises honey.
28. The composition according to embodiment 27, wherein the honey is substantially sourced from *Leptospermum* species.
29. The composition according to embodiment 28, wherein the Leptospermum species is selected from the group consisting of Leptospermum scoparium, Leptospermum polygalifolium, Leptospermum semibaccatum, Leptospermum trinervium, Leptospermum whitei, Leptospermum speciosum, Leptospermum liversidgei and combinations thereof.
30. The composition according to any one of embodiments 1-29, wherein the composition further comprises an ocular lubricant.
31. The composition according to embodiment 30, wherein the lubricant is selected from the group consisting of glucose, glycerol, polyethylene glycol, propylene glycol and combinations thereof.
32. The composition according to embodiment 30, wherein the lubricant is glycerol.
33. A method of treating or inhibiting the development of an infection in a subject, comprising administering a composition according to any one of embodiments 1-32 to the subject.
34. The method according to embodiment 33, wherein the infection is a bacterial or viral infection.
35. The method according to embodiment 34, wherein the infection is a viral infection.
36. The method according to any one of embodiments 33-35, wherein the infection in an acute infection.
37. The method according to any one of embodiments 33-35, wherein the infection is a chronic infection.
38. The method according to any one of embodiments 33-37, wherein the infection is an ophthalmic infection, vaginal infection, nasal infection, ear infection, lung infection, sinus infection, throat infection, skin infection or wound infection.
39. The method according to embodiment 38, wherein the infection is an ophthalmic infection or a nasal infection.
40. A method of treating or inhibiting the development of an inflammatory condition in a subject, comprising administering a composition according to any one of embodiments 1-32 to the subject.
41. The method according to embodiment 40, wherein the inflammatory condition is an ocular condition.
42. The method according to embodiment 41, wherein the ocular condition is blepharitis, dry eye syndrome or corneal oedema.
43. The method according to embodiment 40, wherein the inflammatory condition is a nasal condition.
44. The method according to embodiment 43, wherein the nasal condition is rhinitis.
45. The method according to embodiment 40, wherein the inflammatory condition is sinusitis.
46. The method according to embodiment 40, wherein the inflammatory condition is eczema, contact dermatitis or urticaria.
47. The method according to embodiment 40, wherein the inflammatory condition is an allergic reaction.
48. The method according to embodiment 47, wherein the allergic reaction is associated with an insect bite or an infection.
49. The method according to embodiment 40, wherein the inflammatory condition is associated with an infection.
50. A method of stabilising the pH of a composition comprising honey or a honey analogue, comprising adding gluconic acid or a pharmaceutically acceptable salt or derivative thereof to the composition.
51. The method according to embodiment 50, wherein the method further comprises adding a carrier to the composition before or after adding the gluconic acid or a pharmaceutically acceptable salt or derivative thereof.
52. The method according to embodiment 50 or embodiment 51, wherein the honey or honey analogue is in an amount in the range of from about 2% to about 98% w/w (and all one tenth integer percentages in between).
53. The method according to v 52, wherein the honey or honey analogue is in an amount in the range of from about 2% to about 50% w/w (and all one tenth integer percentages in between).
54. The method according to embodiment 52, wherein the honey or honey analogue is in an amount in the range of from about 2% to about 45% w/w (and all one tenth integer percentages in between).
55. The method according to embodiment 52, wherein the honey or honey analogue is in an amount in the range of from about 7 to about 18% w/w (and all one tenth integer percentages in between).
56. The method according to embodiment 52, wherein the honey or honey analogue is in an amount of about 16.5% w/w.
57. The method according to any one of embodiments 50-56, wherein the gluconic acid or a pharmaceutically acceptable salt or derivative thereof is added in an amount in the range of from about 0.05% to about 10% w/w of the composition (and all one tenth integer percentages in between).
58. The method according to embodiment 57, wherein the gluconic acid or a pharmaceutically acceptable salt or derivative thereof is added in an amount in the range of from about 0.1% to about 5% w/w of the composition (and all one tenth integer percentages in between).
59. The method according to embodiment 57, wherein the gluconic acid or a pharmaceutically acceptable salt or derivative thereof is added in an amount in the range of from about 0.1% to about 3% w/w of the composition (and all one tenth integer percentages in between).
60. The method according to embodiment 57, wherein the gluconic acid or a pharmaceutically acceptable salt or derivative thereof is added in an amount in the range of from about 0.1% to about 1% w/w of the composition (and all one tenth integer percentages in between).
61. The method according to embodiment 57, wherein the gluconic acid or a pharmaceutically acceptable salt or derivative thereof is added in an amount in the range of from about 0.1% to about 0.5% w/w of the composition (and all one tenth integer percentages in between).
62. The method according to embodiment 57, wherein the gluconic acid or a pharmaceutically acceptable salt or derivative thereof is added in an amount of about 0.3% w/w of the composition.
63. The method according to any one of embodiments 50-62, wherein the gluconic acid is in the form of a pharmaceutically acceptable salt.
64. The method according to embodiment 63, wherein the pharmaceutically acceptable salt is selected from the group consisting of sodium gluconate, calcium gluconate, potassium gluconate and magnesium gluconate.
65. The method according to embodiment 63, wherein the pharmaceutically acceptable salt is sodium gluconate.
66. The method according to any one of embodiments 50-65, wherein the method further comprises adding a preservative to the composition before or after adding the gluconic acid or a pharmaceutically acceptable salt or derivative thereof.
67. The method according to embodiment 66, wherein the preservative is selected from the group consisting of benzoic acid, sorbic acid, a flavonoid, a phenolic acid, abscisic acid, ascorbic acid and pharmaceutically acceptable salts and combinations thereof.
68. The method according to embodiment 66, wherein the preservative is benzoic acid or a pharmaceutically acceptable salt thereof.
69. The method according to embodiment 66, wherein the preservative is sodium benzoate.
70. The method according to any one of embodiments 50-69, wherein the pH of the composition is maintained in the range of from about 3.5 to about 4.5 (and all one tenth integers in between).
71. The method according to embodiment 70, wherein the pH of the composition is maintained in the range of from about 3.9 to about 4.1 (and all one tenth integers in between).
72. The method according to embodiment 70 or embodiment 71, wherein the pH of the composition is maintained for a period of at least six months.
73. The method according to embodiment 70 or embodiment 71, wherein the pH of the composition is maintained for a period of at least two years.
74. The method according to any one of embodiments 50-73, wherein the composition is a pharmaceutical composition.
75. The method according to any one of embodiments 50-74, wherein the composition is in the form of a nasal spray, nasal lavage, eye drop, eye spray, eye wash, eye ointment, ear wash, ear drop, throat spray, douche, wound dressing, wound dressing hydrator, wound cleansing solution or lung aspirant.
76. The method according to embodiment 75, wherein the composition is in the form of a nasal spray or an eye drop.
77. The method according to any one of embodiments 51-76, wherein the carrier is an aqueous carrier, glycerol or a combination thereof.
78. The method according to embodiment 77, wherein the aqueous carrier is saline or water.
79. The method according to any one of embodiments 50-78, wherein the composition comprises honey.
80. The method according to embodiment 79, wherein the honey is substantially sourced from *Leptospermum* species.
81. The method according to embodiment 80, wherein the Leptospermum species is selected from the group consisting of Leptospermum scoparium, Leptospermum polygalifolium, Leptospermum semibaccatum, Leptospermum trinervium, Leptospermum whitei, Leptospermum speciosum, Leptospermum liversidgei and combinations thereof.
82. The method according to any one of embodiments 50-81, wherein the method further comprises adding an ocular lubricant to the composition before or after adding the gluconic acid or a pharmaceutically acceptable salt or derivative thereof.
83. The method according to embodiment 82, wherein the lubricant is selected from the group consisting of glucose, glycerol, polyethylene glycol, propylene glycol and combinations thereof.
84. The method according to embodiment 82, wherein the lubricant is glycerol.

## Claims

1. A composition comprising honey or a honey analogue, gluconic acid or a pharmaceutically acceptable salt or derivative thereof and a pharmaceutically acceptable carrier.

2. The composition according to claim 1, wherein the honey or honey analogue is in an amount in the range of from about 2% to about 98% w/w, preferably wherein the honey or honey analogue is in an amount in the range of from about 2% to about 50% w/w, preferably wherein the honey or honey analogue is in an amount in the range of from about 2% to about 45% w/w, preferably wherein the honey or honey analogue is in an amount in the range of from about 7% to about 18% w/w, more preferably wherein the honey or honey analogue is in an amount of about 16.5% w/w.

3. The composition according to claim 1 or claim 2, wherein the gluconic acid or a pharmaceutically acceptable salt or derivative thereof is in an amount in the range of from about 0.05% to about 10% w/w, preferably wherein the gluconic acid or a pharmaceutically acceptable salt or derivative thereof is in an amount in the range of from about 0.1% to about 5% w/w, preferably wherein the gluconic acid or a pharmaceutically acceptable salt or derivative thereof is in an amount in the range of from about 0.1% to about 3% w/w, preferably wherein the gluconic acid or a pharmaceutically acceptable salt or derivative thereof is in an amount in the range of from about 0.1% to about 1% w/w, preferably wherein the gluconic acid or a pharmaceutically acceptable salt or derivative thereof is in an amount in the range of from about 0.1% to about 0.5% w/w, more preferably wherein the gluconic acid or a pharmaceutically acceptable salt or derivative thereof is in an amount of about 0.3% w/w.

4. The composition according to any one of claims 1-3, wherein the gluconic acid is in the form of a pharmaceutically acceptable salt, preferably wherein the pharmaceutically acceptable salt is selected from the group consisting of sodium gluconate, calcium gluconate, potassium gluconate, magnesium gluconate, zinc gluconate, copper gluconate and ferrous gluconate, preferably wherein the pharmaceutically acceptable salt is sodium gluconate.

5. The composition according to any one of claims 1-4, wherein the composition further comprises a preservative, preferably wherein the preservative is selected from the group consisting of benzoic acid, sorbic acid, a flavonoid, a phenolic acid, abscisic acid, ascorbic acid and pharmaceutically acceptable salts and combinations thereof, preferably wherein the preservative is benzoic acid or a pharmaceutically acceptable salt thereof, more preferably wherein the preservative is sodium benzoate.

6. The composition according to any one of claims 1-5, wherein the pH of the composition is in the range of from about 3.5 to about 4.5, preferably in the range of from about 3.9 to about 4.1.

7. The composition according to any one of claims 1-6, wherein the composition is in the form of a nasal spray, eye drop, nasal lavage, eye spray, eye wash, eye ointment, ear wash, ear drop, throat spray, douche, wound dressing, wound dressing hydrator, wound cleansing solution or lung aspirant, preferably a nasal spray or an eye drop.

8. The composition according to any one of claims 1-7, wherein the carrier is an aqueous carrier, glycerol or a combination thereof, preferably wherein the aqueous carrier is saline or water.

9. The composition according to any one of claims 1-8, wherein the composition further comprises an ocular lubricant, preferably wherein the lubricant is selected from the group consisting of glycerol, glucose, polyethylene glycol, propylene glycol and combinations thereof, preferably wherein the lubricant is glycerol.

10. A composition according to any one of claims 1-9 for use in treating or inhibiting the development of an infection or an inflammatory condition in a subject.

11. The composition for use according to claim 10, wherein the infection is a bacterial or viral infection, preferably a viral infection, preferably wherein the infection is an ophthalmic infection, nasal infection, vaginal infection, ear infection, lung infection, sinus infection, throat infection, skin infection or wound infection, preferably an ophthalmic infection or a nasal infection.

12. The composition for use according to claim 10, wherein the inflammatory condition is an ocular condition such as blepharitis, dry eye syndrome or corneal oedema; a nasal condition such as rhinitis; sinusitis; eczema; contact dermatitis; urticaria; or an allergic reaction, such as an allergic reaction associated with an insect bite or an infection.

13. A method for stabilising the pH of a composition comprising honey or a honey analogue, wherein the method comprises adding gluconic acid or a pharmaceutically acceptable salt or derivative thereof to the composition.

14. Use of gluconic acid or a pharmaceutically acceptable salt or a derivative thereof for stabilizing the pH of a composition comprising honey or a honey analogue.

15. The method according to claim 13 or the use according to claim 14, wherein the honey or honey analogue is in an amount in the range of from about 2% to about 98% w/w, preferably wherein the honey or honey analogue is in an amount in the range of from about 2% to about 50% w/w, preferably wherein the honey or honey analogue is in an amount in the range of from about 2% to about 45% w/w, preferably wherein the honey or honey analogue is in an amount in the range of from about 7 to about 18% w/w, preferably wherein the honey or honey analogue is in an amount of about 16.5% w/w; and/or the gluconic acid or a pharmaceutically acceptable salt or derivative thereof is added in an amount in the range of from about 0.05% to about 10% w/w of the composition, preferably wherein the gluconic acid or a pharmaceutically acceptable salt or derivative thereof is added in an amount in the range of from about 0.1% to about 5% w/w of the composition, preferably wherein the gluconic acid or a pharmaceutically acceptable salt or derivative thereof is added in an amount in the range of from about 0.1% to about 3% w/w of the composition, preferably wherein the gluconic acid or a pharmaceutically acceptable salt or derivative thereof is added in an amount in the range of from about 0.1% to about 1% w/w of the composition, preferably wherein the gluconic acid or a pharmaceutically acceptable salt or derivative thereof is added in an amount in the range of from about 0.1% to about 0.5% w/w of the composition, preferably wherein the gluconic acid or a pharmaceutically acceptable salt or derivative thereof is added in an amount of about 0.3% w/w of the composition.
